# EUROPEAN PATENT SPECIFICATION

(11) **EP 2 202 309 B1**
(45) Date of publication and mention of the grant of the patent: **16.04.2014**
(21) Application number: 09252749.8
(22) Date of filing: 08.12.2009
(51) Int. Cl.: C12N 15/113, C12N 5/10

(54) **Efficient method for nuclear reprogramming**
Effiziente Methode zum nuklearen Reprogrammieren
Méthode éfficace pour la reprogrammation nucléaire

(30) Priority: 08.12.2008 JP 2008312745; 26.12.2008 JP 2008335129
(43) Date of publication of application: 30.06.2010
(73) Proprietor: Kyoto University, Kyoto-shi, Kyoto 606-8501 (JP)
(72) Inventor: Yamanaka, Shinya, Kyoto-shi Kyoto 606-8501 (JP); Koyanagi, Michiyo, Kyoto-shi Kyoto 606-8501 (JP)
(74) Representative: Woods, Geoffrey Corlett

(56) References cited:
- US-A1- 2008 293 143
- CARD DEBORAH A GREER ET AL: "Oct4/Sox2-regulated miR-302 targets cyclin D1 in human embryonic stem cells." MOLECULAR AND CELLULAR BIOLOGY OCT 2008 LNKD- PUBMED:18710938, vol. 28, no. 20, October 2008 (2008-10), pages 6426-6438, XP002577530 ISSN: 1098-5549
- LIN SHI-LUNG ET AL: "Role of mir-302 MicroRNA Family in Stem Cell Pluripotency and Renewal" 1 August 2008 (2008-08-01), CURRENT PERSPECTIVES IN MICRORNAS (MIRNA), SPRINGER NETHERLANDS LNKD- DOI:10.1007/978-1-4020-8533-8, PAGE(S) 167 - 185 , XP009121067 ISBN: 9781402085321 [retrieved on 2008-09-12] * the whole document *
- LIN SHI-LUNG ET AL: "Mir-302 reprograms human skin cancer cells into a pluripotent ES-cell-like state" RNA, COLD SPRING HARBOR LABORATORY PRESS, US LNKD- DOI:10.1261/RNA.1162708, vol. 14, no. 10, 28 August 2008 (2008-08-28), pages 2115-2124, XP009108022 ISSN: 1355-8382
- TAY YVONNE ET AL: "MicroRNAs to Nanog, Oct4 and Sox2 coding regions modulate embryonic stem cell differentiation." NATURE 23 OCT 2008 LNKD- PUBMED:18806776, vol. 455, no. 7216, 23 October 2008 (2008-10-23), pages 1124-1128, XP002577531 ISSN: 1476-4687
- SUH MI-RA ET AL: "Human embryonic stem cells express a unique set of microRNAs" DEVELOPMENTAL BIOLOGY, ACADEMIC PRESS, NEW YORK, NY, US, vol. 270, no. 2, 6 May 2004 (2004-05-06), pages 488-498, XP002404396 ISSN: 0012-1606
- LAURENT LOUISE C ET AL: "Comprehensive microRNA profiling reveals a unique human embryonic stem cell signature dominated by a single seed sequence." STEM CELLS (DAYTON, OHIO) JUN 2008 LNKD- PUBMED:18403753, vol. 26, no. 6, June 2008 (2008-06), pages 1506-1516, XP002577532 ISSN: 1549-4918
- ZHANG BAOHONG ET AL: "MicroRNA: A new player in stem cells" JOURNAL OF CELLULAR PHYSIOLOGY, WILEY LISS, NEW YORK, NY, US LNKD- DOI:10.1002/JCP.20713, vol. 209, no. 2, 1 November 2006 (2006-11-01), pages 266-269, XP002540098 ISSN: 0021-9541
- MARSON ALEXANDER ET AL: "Connecting microRNA genes to the core transcriptional regulatory circuitry of embryonic stem cells." CELL 8 AUG 2008 LNKD- PUBMED:18692474, vol. 134, no. 3, 8 August 2008 (2008-08-08), pages 521-533, XP002577533 ISSN: 1097-4172
- JUDSON ROBERT L ET AL: "Embryonic stem cell-specific microRNAs promote induced pluripotency." NATURE BIOTECHNOLOGY MAY 2009 LNKD- PUBMED:19363475, vol. 27, no. 5, 12 April 2009 (2009-04-12) , pages 459-461, XP002577534 ISSN: 1546-1696
- Sabiosciences: "Induced pluripotent stem cells - Quick Facts", Technical notes, 1 January 2009 (2009-01-01), XP055060058, Retrieved from the Internet: URL:http://sabiosciences.com/manuals/iPSCs temCELLS.pdf [retrieved on 2013-04-17]

### SEQUENCE LISTING

<110> Kyoto University
<120> Efficient Method for Nuclear Reprogramming
<130> PH-4191EP
<150> JP 2008-312745
   <151> 2008-12-08
<150> JP 2008-335129
   <151> 2008-12-26
<160> 14
<170> PatentIn version 3.4
<210> 1
   <211> 84
   <212> RNA
   <213> Mus musculus
<400> 1
   uuccauauag ccauacucaa aauggaggcc cuaucuaagc uuuuaagugg aaagugcuuc 60
   ccuuuugugu guugccaugu ggag 84
<210> 2
   <211> 69
   <212> RNA
   <213> Mus musculus
<400> 2
   ggugagacuc aaaugugggg cacacuucug gacuguacau agaaagugcu acuacuuuug 60
   agucucucc 69
<210> 3
   <211> 67
<212> RNA
   <213> Homo sapiens
<400> 3
   gugggccuca aauguggagc acuauucuga uguccaagug gaaagugcug cgacauuuga 60
   gcgucac 67
<210> 4
   <211> 69
   <212> RNA
   <213> Homo sapiens
<400> 4
   gggauacuca aaaugggggc gcuuuccuuu uugucuguac ugggaagugc uucgauuuug 60
   ggguguccc 69
<210> 5
   <211> 73
   <212> RNA
   <213> Homo sapiens
<400> 5
   gcucccuuca acuuuaacau ggaagugcuu ucugugacuu uaaaaguaag ugcuuccaug 60
   uuuuaguagg agu 73
<210> 6
   <211> 68
   <212> RNA
   <213> Homo sapiens
<400> 6
   ccuuugcuuu aacauggggg uaccugcugu gugaaacaaa aguaagugcu uccauguuuc 60
   aguggagg 68
<210> 7
   <211> 69
   <212> RNA
   <213> Homo sapiens
<400> 7
   ccaccacuua aacguggaug uacuugcuuu gaaacuaaag aaguaagugc uuccauguuu 60
   uggugaugg 69
<210> 8
   <211> 68
   <212> RNA
   <213> Homo sapiens
<400> 8
   ccucuacuuu aacauggagg cacuugcugu gacaugacaa aaauaagugc uuccauguuu 60
   gagugugg 68
<210> 9
   <211> 68
   <212> RNA
   <213> Homo sapiens
<400> 9
   ccauuacugu ugcuaauaug caacucuguu gaauauaaau uggaauugca cuuuagcaau 60
   ggugaugg 68
<210> 10
   <211> 87
   <212> RNA
   <213> Homo sapiens
<400> 10
   ucucaggcug ucguccucua gagggaagca cuuucuguug ucugaaagaa aagaaagugc 60
   uuccuuuuag aggguuaccg uuugaga 87
<210> 11
   <211> 82
   <212> RNA
   <213> Mus musculus
<400> 11
   ccuauguagc ggccaucaaa guggaggccc ucucuugagc cugaaugaga aagugcuucc 60
   acuuugugug ccacugcaug gg 82
<210> 12
   <211> 820
   <212> RNA
   <213> Homo sapiens
<400> 12
   guuuucuuuc uccucagcuc uaaauacucu gaaguccaaa gaaguuguau guuggguggg 60
   cucccuucaa cuuuaacaug gaagugcuuu cugugacuuu aaaaguaagu gcuuccaugu 120
   uuuaguagga gugaauccaa uuuacuucuc caaaauagaa cacgcuaacc ucauuugaag 180
   ggauccccuu ugcuuuaaca uggggguacc ugcuguguga aacaaaagua agugcuucca 240
   uguuucagug gaggugucuc caagccagca caccuuuugu uacaaaauuu uuuuguuauu 300
   guguuuuaag guuacuaagc uuguuacagg uuaaaggauu cuaacuuuuu ccaagacugg 360
   gcuccccacc acuuaaacgu ggauguacuu gcuuugaaac uaaagaagua agugcuucca 420
   uguuuuggug augguaaguc uuccuuuuac auuuuuauua uuuuuuuaga aaauaacuuu 480
   auuguauuga ccgcagcuca uauauuuaag cuuuauuuug uauuuuuaca ucuguuaagg 540
   ggcccccucu acuuuaacau ggaggcacuu gcugugacau gacaaaaaua agugcuucca 600
   uguuugagug uggugguucc uaccuaauca gcaauugcgu uaacgcccac acugugugca 660
   guucuuggcu acaggccauu acuguugcua auaugcaacu cuguugaaua uaaauuggaa 720
   uugcacuuua gcaaugguga uggauuguua agccaaugac agaauuuaaa ccacagacuu 780
   acuuugauag cacucuuaau gguauaacuu cuucucccau 820
<210> 13
   <211> 83
   <212> RNA
   <213> Mus musculus
<400> 13
   cucaucuugc gguacucaaa cuaugggggc acuuuuuuuu uucuuuaaaa agugccgccu 60
   aguuuuaagc cccgccgguu gag 83
<210> 14
   <211> 965
   <212> RNA
   <213> Homo sapiens
<400> 14
   cuucaacagc ucaucaaggg cuacucucca ccuccuugcu uaaaagccuc uucugauggg 60
   uaagugcuuc cacuugcgau cgccgccuug ccgcaucccc ucagccugug gcacucaaac 120
   ugugggggca cuuucugcuc ucuggugaaa gugccgccau cuuuugagug uuaccgcuug 180
   agaagacuca accugcggag aagauaccau uuugauuggg ugagggggcg gguagcagga 240
   uggcccuaga cccugccuau ggccguuucc ucgugauaua aauuucuugg ccggggcucu 300
   ugcagaugga gcugcucacc cugugggccu caaaugugga gcacuauucu gauguccaag 360
   uggaaagugc ugcgacauuu gagcgucacc ggugacgccc auaucaacgg augccgugga 420
   gcucggucuu cugcaggaac uaaagagccu gugguuucga uucccagccg gaaacugucu 480
   uggguacagg ucccuuacag cgucuggcug uaauggcucc ggaaaaccug gggaagggaa 540
   gggggccucc ugggcucuca ccugacacaa cuaagggaau cuggguuaga uggugaaagg 600
   aagagaaggu ucagagggcg gcugugcgcc ugcgccgggc gcggcggcuc acaccuguaa 660
   ucccagcacg uugggaggcc gaggcaggcg gaucacgagg ucaggagauc gagaccaucc 720
   uggcuaacac ggugaaaccc cgucucuacu aaaaauacaa aaaaugagcc gggcguggug 780
   gcgggcgccu guagucccag cuacuuggga ggcugaggca ggagaauggc gugaacccgg 840
   gaggcggagu uugcagugag ccgagaucgc gccaccgcac uccagccugg gcgacagagc 900
   aagacucauu cucaaagaaa aaaaaaaaaa aaaacagcag cugugaccaa ggggcuguau 960
   gcaca 965

## Description

### TECHNICAL FIELD

The present invention relates to a method of reprogramming differentiated somatic cells to efficiently produce induced pluripotent stem cells.

### BACKGROUND ART

Embryonic stem cells (ES cells) are stem cells that have been established from human or mouse early embryos, and such stem cells can be cultured for a long period of time while maintaining the ability to differentiate into all types of cells *in vivo*. With the utilization of such properties, human ES cells are expected as resources for cell transplantation for treating many diseases, such as Parkinson's disease, juvenile diabetes, or leukemia. However, ES cell transplantation disadvantageously induces rejection, as with the case of organ transplantation. Also, there are many adverse opinions regarding the use of ES cells established by destroying human embryos from an ethical point of view.

If differentiated somatic cells of a patient are used to induce dedifferentiation and cells having pluripotency and proliferation capacity that are similar to those of ES cells can be established (such cells are referred to as "induced pluripotent stem cells" or "iPS cells" herein and these cells are occasionally referred to as "artificially induced pluripotent stem cells," "embryonic stem cell-like cells," or "ES-like cells"), use of such cells as ideal pluripotent cells that will not be rejected and do not present ethical concerns can be expected. It has been reported recent years that such iPS cells can be produced from mouse and human differentiated somatic cells, and such report has created a great sensation (WO 2007/69666; Cell, 126, pp. 663-676, 2006; Cell, 131, pp. 861-872, 2007; Science, 318, pp. 1917-1920, 2007; Nature, 451, pp. 141-147, 2008). Such techniques involve a step of introducing a plurality of given factors (4 factors; i.e., Oct3/4, Sox2, Klf4, and c-Myc, are used in Cell, 126, pp. 1-14, 2006) into somatic cells to perform reprogramming, and such factors are introduced with the use of virus vectors such as retrovirus or lentivirus vectors. Nuclear reprogramming techniques via gene transfer that have been reported in the past, however, are low in terms of efficiency, and the amount of induced pluripotent stem cells that can be produced is very small. When reprogramming is performed by introducing 3 factors (i.e., Oct3/4, Sox2, and Klf4) except for c-Myc (which may involve tumorigenesis) into somatic cells of a chimera mouse produced from tissues differentiated from induced pluripotent stem cells or from induced pluripotent stem cells, in particular, the efficiency of obtaining induced pluripotent stem cells is disadvantageously very low.

Meanwhile, it is known that a variety of small RNAs are expressed in cells. An example of small RNA is an RNA molecule comprising about 18 to 25 nucleotides which is cut out with Dicer, a double-stranded RNA-specific RNase. Small RNAs are mainly classified into siRNA (small interfering RNA) and miRNA (microRNA, hereinafter abbreviated as "miRNA"). Small RNAs are known to function as guide molecules to find a target during the process of inhibition of translation, mRNA degradation, or chromatin structural change via the RNA interference (RNAi)/miRNA molecular mechanism It is also known that small RNA plays a key role in regulation of the course of development (see, for example, Jikken Igaku ("Experimental Medicine"), 24, pp. 814-819, 2006; microRNA Jikken Purotokoru ("microRNA Experimental Protocols"), pp. 20-35, 2008, Yodosha Co., Ltd. for review).

Regarding the relationship between embryonic stem cells (ES cells) and miRNA, it has been reported that an miRNA cluster containing several types of miRNAs is expressed in an ES-cell-specific manner in mouse ES cells (Developmental cell, 5, pp. 351-358, 2003). It has also been reported that miRNA-295 is involved in methylation of DNA by inhibiting the expression of Rb12, which is a family member of the cancer suppressor gene Rb, and elevating expression levels of methylase (Nature Structural & Molecular Biology, 15, pp. 259-267, 2008; Nature Structural & Molecular Biology, 15, pp. pp. 268-279, 2008). However, it was not known in the past that small RNA has some function in nuclear reprogramming of somatic cells.

### DISCLOSURE OF THE INVENTION

It is an object of the present invention to provide a means for efficiently producing induced pluripotent stem cells when reprogramming somatic cells to produce induced pluripotent stem cells.

The present inventors have conducted intensive studies in order to achieve the above-described object. As a result, the present inventors discovered that induced pluripotent stem cells can be efficiently produced by introducing genes, which are inducible nuclear reprogramming, into somatic cells in the presence of a certain miRNA(s).

Specifically, in the first aspect the present invention provides a method for preparing an induced pluripotent stem cell from a somatic cell, comprising a step of nuclear reprogramming of the somatic cell with an exogenous nuclear reprogramming factor(s) in the presence of at least one exogenous miRNA, wherein:
(a) the nuclear reprogramming factor comprises no Sox family member and
   (i) Oct3/4;
   (ii) Oct3/4 and a Klf family member selected from Klfl, Klf2, Klf4 and Klf5; or
   (iii) Oct3/4 and Nanog,
   and the miRNA is a miRNA of the hsa-miR-302-367 cluster or a mature miRNA thereof; or
(b) the nuclear reprogramming factor comprises Oct3/4, a Klf family member selected from Klf1, Klf2, Klf4 and Klf5, a Myc family member selected from c-Myc, N-Myc and L-Myc and no Sox family member, and the miRNA is selected from mmu-miR-295/295* the hsa-miR-302-367 cluster and mature miRNAs thereof; or
(c) the nuclear reprogramming factor comprises Oct3/4, Sox2 and a Klf family member selected from Klf1, Klf2, Klf4 and Klf5, and the miRNA is one or more miRNAs selected from the group consisting of:
   - hsa-miR-372;
   - hsa-miR-373 or hsa-miR-373/373*;
   - hsa-miR-371-373 cluster;
   - hsa-miR-302b or hsa-miR-302b/302b*;
   - hsa-miR-302-367 cluster;
   - hsa-miR-520c or hsa-miR-520c-5p/520c-3p;
   - mmu-miR-291a or mmu-miR-291a-5p/291a-3p;
   - mmu-miR-294 or mmu-miR-294/294*;
   - mmu-miR-295 or mmu-miR-295/295 *; and
   - mature miRNAs thereof,
where the symbols indicate the miRBase database registration names.

In the second aspect, the present invention provides a method for increasing the nuclear reprogramming efficiency for inducing an induced pluripotent stem cell from a somatic cell, which method comprises subjecting the somatic cell to nuclear reprogramming with an exogenous nuclear reprogramming factor(s) in the presence of at least one exogenous miRNA, wherein:
(a) the nuclear reprogramming factor comprises no Sox family member and
   (i) Oct3/4;
   (ii) Oct3/4 and a Klf family member selected from Klf1, Klf2, Klf4 and Klf5; or
   (iii) Oct3/4 and Nanog,
   and the miRNA is the hsa-miR-302-367 cluster or a mature miRNA thereof; or
(b) the nuclear reprogramming factor comprises Oct3/4, a Klf family member selected from Klf1, Klf2, Klf4 and Klf5, a Myc family member selected from c-Myc, N-Myc and L-Myc and no Sox family member, and the miRNA is selected from mmu-miR-295/295* , the hsa-miR-302-367 cluster and mature miRNAs thereof; or
(c) the nuclear reprogramming factor comprises Oct3/4, Sox2 and a Klf family member selected from Klf1, Klf2, Klf4 and Klf5, and the miRNA is one or more miRNAs selected from the group consisting of:
   - hsa-miR-372;
   - hsa-miR-373 or hsa-miR-373/373*;
   - hsa-miR-371-373 cluster;
   - hsa-miR-302b or hsa-miR-302b/302b*;
   - hsa-miR-302-367 cluster;
   - hsa-miR-520c or hsa-miR-520c-5p/520c-3p;
   - mmu-miR-291 a or mmu-miR-291a-5p/291a-3p;
   - mmu-miR-294 or mmu-miR-294/294*;
   - mmu-miR-295 or mmu-miR-295/295*; and
   - mature miRNAs thereof,
where the symbols indicate the miRBase database registration names. The term "a high nuclear reprogramming efficiency" means that the number of induced pluripotent stem cell colonies in the presence of miRNA is larger than that in the absence of miRNA, preferably at least 1.5 times, and more preferably at least 3 times larger.

According to the present invention, the miRNA is the one that is expressed at a higher level in embryonic stem cells than in somatic cells, and preferably the miRNA is the one that is expressed in embryonic stem cells but is not expressed or is almost not expressed in somatic cells.

According to a preferred embodiment of the above invention, the Klf family member is Klf4, and/or the Myc family member is c-Myc.

Preferable combinations of nuclear reprogramming factors are: (a) Oct3/4; (b) Oct3/4 and Klf4; (c) Oct3/4 and Nanog; or (d) Oct3/4, Klf4, and c-Myc.

According to another preferred embodiment, the method of the present invention may comprise introducing a vector containing a DNA(s) encoding a nuclear reprogramming factor(s) and/or at least one miRNA, into the somatic cells.

Alternatively, the method may comprise introducing a vector comprising a DNA(s) encoding a nuclear reprogramming factor(s) and/or a vector comprising a DNA encoding at least one miRNA or precursor RNA thereof, into the somatic cells.

Somatic cells are from mammalian animals, preferably primates, rodents, ungulates, and pet animals, more preferably humans. Somatic cells may be from any of adults, neonates, and fetuses. Also, somatic cells may be cells derived from any tissue, hemopoietic cells, lymphatic cells, or tissue stem cells (somatic stem cells) for example. Further, somatic cells may be from healthy individuals or individuals with diseases.

The miRNA may be one or more miRNAs contained in one or more RNA sequences selected from SEQ ID NOS: 1 to 14, and preferably from SEQ ID NOS: 1 to 5, 10 to 12, and 14, as shown in the Sequence Listing. In the above-described registration names, the symbols "hsa" and "mmu" represent "Homo sapiens" and "Mus *musculus*," respectively.

According to an embodiment of the present invention, the miRNA has a nucleotide sequence comprising 18 to 25 nucleotides. The nucleotide size of the miRNA is preferably 20 to 25 nucleotides, and more preferably 21 to 23 nucleotides.

The present invention further provides a kit for preparing induced pluripotent stem cells from somatic cells comprising any of the following combinations of components, (a) to (d):
(a) a combination of a miRNA, or a vector encoding the miRNA, with:
   (i) Oct3/4, or a vector containing DNA encoding Oct3/4; or
   (ii) Oct3/4, or a vector containing DNA encoding Oct3/4, and a Klf family member selected from Klf1, Klf2, Klf4 and Klf5, or a vector containing DNA encoding the Klf family member; or
   (iii) Oct3/4, or a vector containing DNA encoding Oct3/4, and Nanog, or a vector containing DNA encoding Nanog;
   wherein the miRNA is the hsa-miR-302-367 cluster or mature miRNA(s) thereof
   comprising 18 to 25 nucleotides; or
(b) a combination of a miRNA, or a vector encoding the miRNA, with Oct3/4, or a vector containing DNA encoding Oct3/4, a Klf family member selected from Klf1, Klf2, Klf4 and Klf5, or a vector containing DNA encoding the Klf family member, and an Myc family member selected from c-Myc, N-Myc and L-Myc, or a vector containing DNA encoding the Myc family member, wherein the miRNA is at least one miRNA selected from the group consisting of: mmu-miR-295/295*, the hsa-miR-302-367 cluster and mature miRNAs thereof comprising 18 to 25 nucleotides; or
(c) a combination of a miRNA, or a vector encoding the miRNA, with Oct3/4, or a vector containing DNA encoding Oct3/4, a Klf family member selected from Klf1, Klf2, Klf4 and Klf5, or a vector containing DNA encoding the Klf family member, and Sox2, or a vector containing DNA encoding Sox2, wherein the miRNA is one or more miRNAs selected from the group consisting of:
   - hsa-miR-372;
   - hsa-miR-373 or hsa-miR-373/373*;
   - hsa-miR-371-373 cluster;
   - hsa-miR-302b or hsa-miR-302b/302b*;
   - hsa-miR-302-367 cluster;
   - hsa-miR-520c or hsa-miR-520c-5p/520c-3p;
   - mmu-miR-291 a or mmu-miR-291a-5p/291a-3p;
   - mmu-miR-294 or mmu-miR-294/294*;
   - mmu-miR-295 or mmu-miR-295/295*; and
   - mature miRNAs thereof comprising 18 to 25 nucleotides; or
(d) a combination of any of (a)(i) to (iii), (b) and (c) above,
where the symbols indicate the miRBase database registration names. According to a preferred embodiment, the Klf family member is Klf4, and the Myc family member is c-Myc.

### BRIEF DESCRIPTION OF THE DRAWINGS

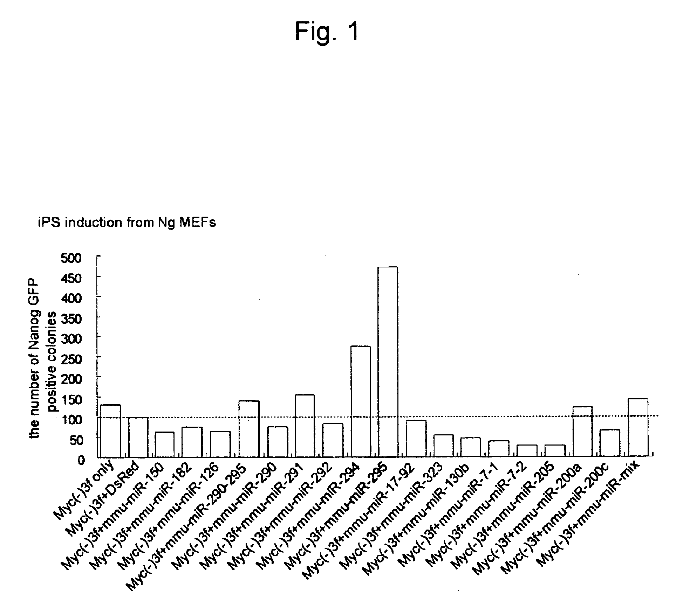

Fig. 1 shows the results of confirmation on the production efficiency of induced pluripotent stem cells through induction of nuclear reprogramming in mouse embryonic fibroblasts with a combination of three genes i.e. Oct3/4, Klf4, and Sox2, (this combination is represented as "3f," and "c-Myc(-)" means that c-Myc was omitted from the combination of four genes i.e. Oct3/4, Klf4, Sox2 and c-Myc, which is highly efficient for nuclear reprogramming), in the presence of a miRNA. 3f+DsRed represents the combination where DsRed (i.e., the *Discosoma sp.* red fluorescent protein) was added as a control to the combination of the aforementioned three genes.

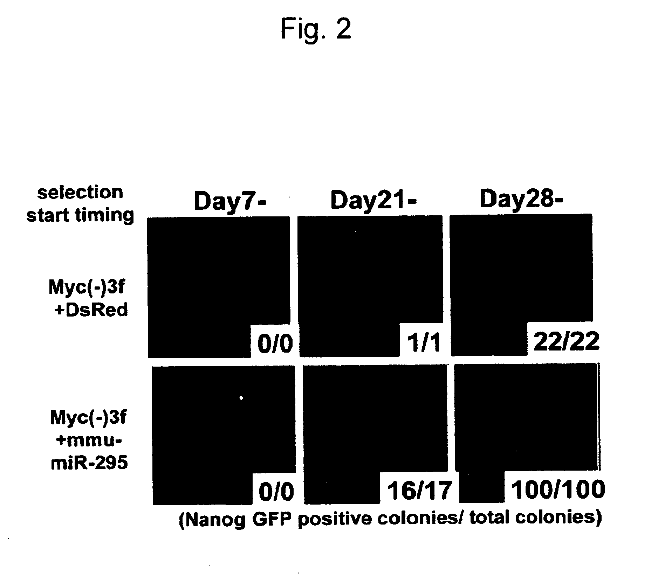

Fig. 2 shows the production efficiency of induced pluripotent stem cells. The top panel shows the results for a combination of three genes Oct3/4, Klf4 and Sox2 (the combination of three genes in which c-Myc was omitted from the combination of four genes) and DsRed as a control. The bottom panel shows the results for induction of nuclear reprogramming in mouse tail tip fibroblasts (TTFs) with the combination of three genes: Oct3/4, Klf4, and Sox2, in the presence of mmu-miR-295. The numerals in the figure indicate the number of Nanog GFP positive colonies/the number of total colonies.

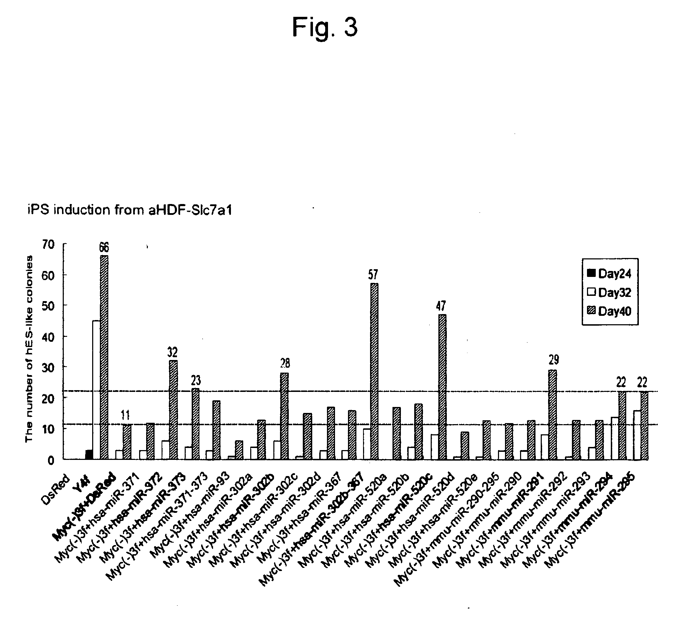

Fig. 3 shows the results of confirmation on the production efficiency of induced pluripotent stem cells through induction of nuclear reprogramming in adult human skin cells with the combination of three genes: Oct3/4, Klf4, and Sox2 (Myc(-)3f: a combination of three genes in which c-Myc was omitted from the combination of four genes: Oct3/4, Klf4, Sox2, and c-Myc), or the combination of four genes: Oct3/4, Klf4, Sox2, and c-Myc (Y4f), in the presence of various miRNAs.

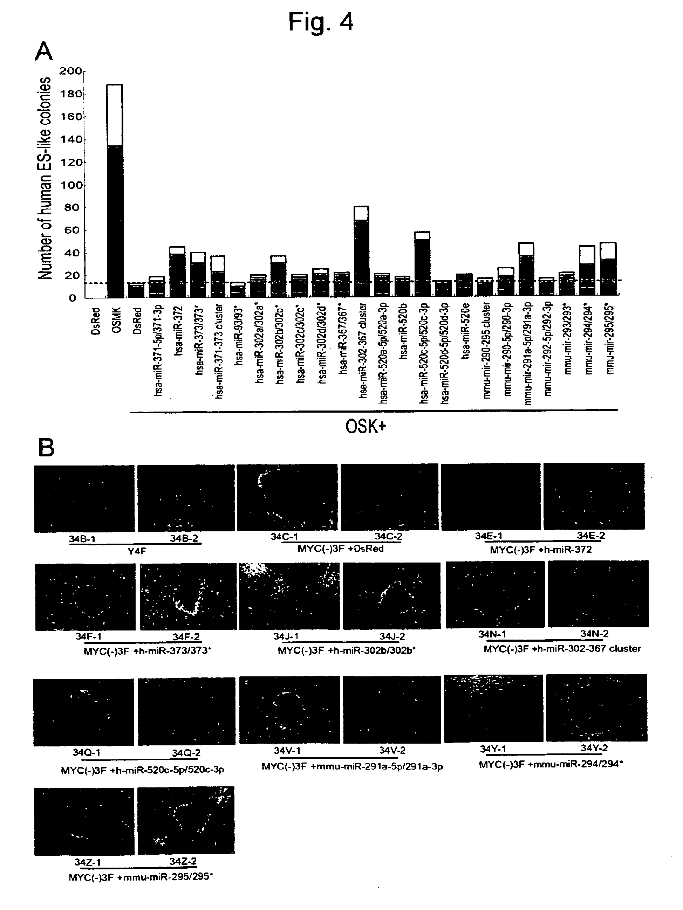

Fig. 4 shows the results of ES cell-like colony formation resulting from transduction with four genes (OCT3/4, SOX2, KLF4, and c-MYC (OSMK)) or three genes (OCT3/4, SOX2, and KLF4) in the presence of various miRNAs. Fig. 4A shows human ES cell-like colonies obtained by transduction with four genes (OSMK) or three genes (c-MYC(-))+miRNAs (OSK+). Fig. 4B shows the morphology of ES cell-like colonies counted in Fig. 4A.

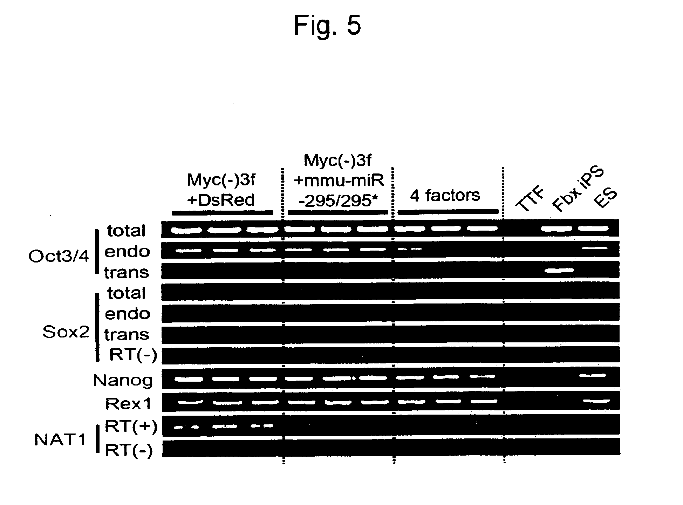

Fig. 5 shows the results of RT-PCR analysis of ES cell marker expression in iPS cells prepared by nuclear reprogramming of mouse tail tip fibroblasts (TTF) with four genes (Oct3/4, Sox2, c-Myc, and Klf4) and with three genes (Oct3/4, Sox2, and Klf4; i.e., "Myc(-)3f") + mmu-miR-295/295^{*} or DsRed.

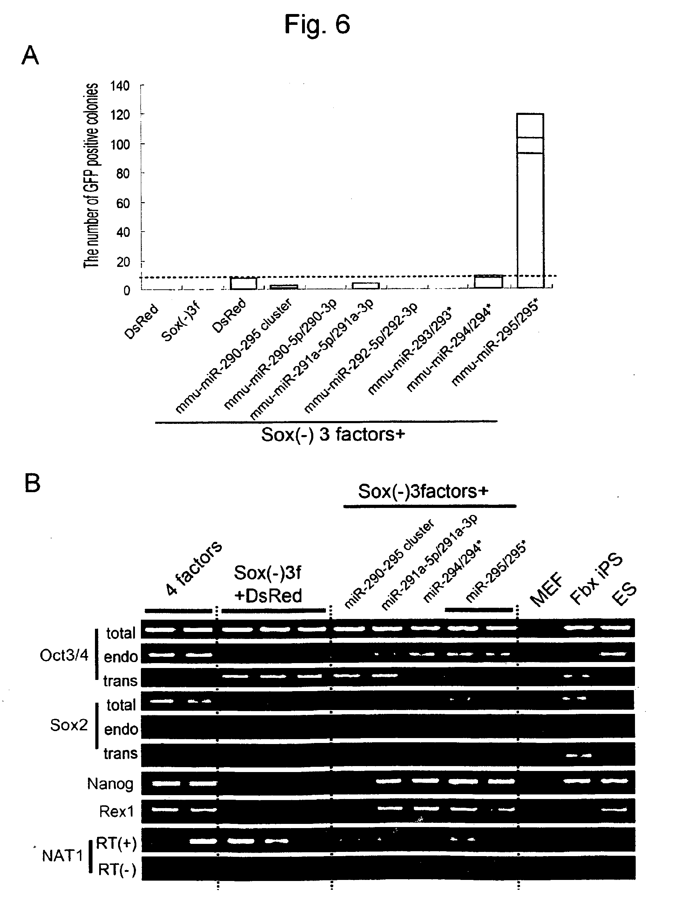

Fig. 6 shows the results of MEF cells infected with three genes (Oct3/4, c-MycWT, and Klf4; i.e., "Sox(-)") + mmu-miR-290-295 cluster, 290-5p/290-3p (mmu-miR-290), 291a-5p/291a-3p (mmu-miR-291a), 292-5p/292-3p (mmu-miR-292), 293/293^{*} (mmu-miR-293), 294/294^{*} (mmu-miR-294), or 295/295^{*} (mmu-miR-295). Fig. 6A shows the number of Nanog GFP positive colonies. Fig. 6B shows expression of ES cell marker genes in iPS cells checked by RT-PCR.

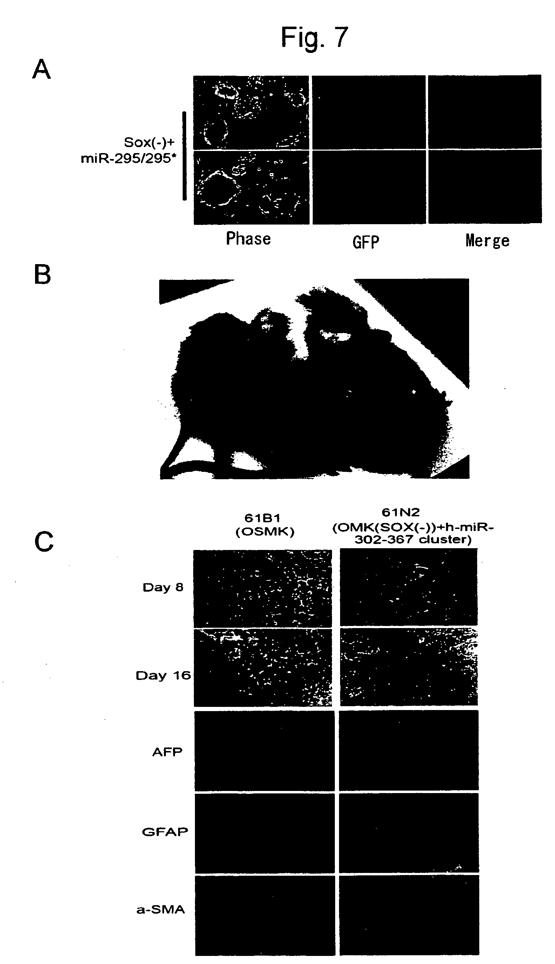

Fig. 7 shows the results of iPS cell induction with Fb-Ng MEFs (Fbx15-βgeo/Nanog-IRES-Puro^{r} reporter mouse-derived MEF) that overexpresses Oct3/4, c-Myc, and Klf4 ("Sox(-)") + mmu-miR-295/295^{*} or hsa-miR-302-367 cluster miRNA. Fig. 7A shows cell morphology of MEFs transduced with Oct3/4, c-Myc, and Klf4 ("Sox(-)") + mmu-miR-295/295^{*}. Fig. 7B shows a chimera mouse derived from iPS cells induced with Sox(-)3f + mmu-miR-295/295*. Fig. 7C shows embryoid body (EB)-mediated *in vitro* differentiation by human iPS cells. Human iPS cells (61B1, 61N2) were established embryoid body (EB) by transduction of four genes (OCT3/4, KLF4, SOX2, and c-MYC; i.e., "OSMK") or three genes (OCT3/4, KLF4, and c-MYC; i.e., "OMK(SOX(-))") in the presence of has-miR-302-367 cluster miRNA. After culturing for 16 days, immunohistochemistry analysis was performed in the cells by using an antibody against each of α-fetoprotein (AFP) which is a differentiation marker for endodernial cells, α-smooth muscle actin (α-SMA) which is a differentiation marker for mesodermal cells, and GFAP (DAKO) which is a differentiation marker for ectodermal cells. Nuclei were stained with Hoechst 33342 (Invitrogen).

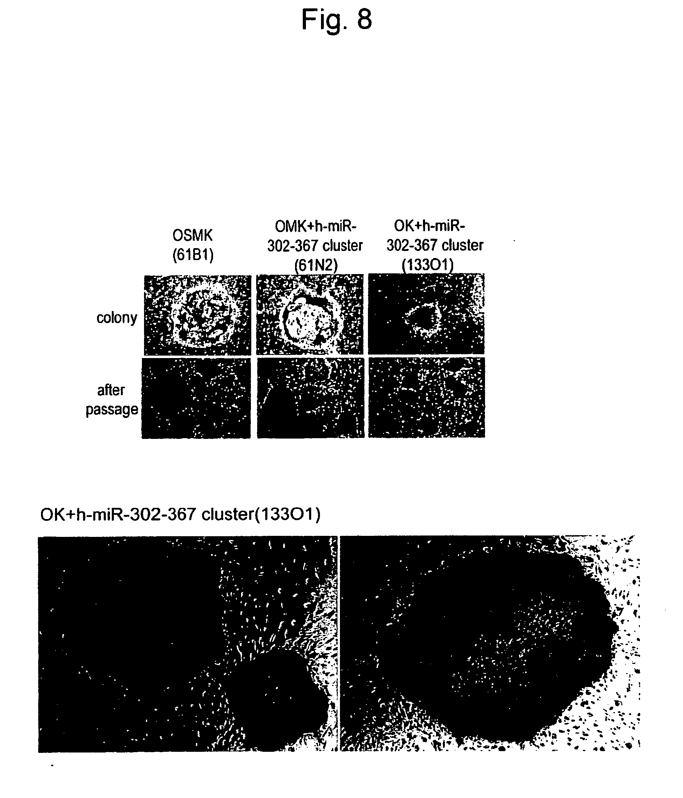

Fig. 8 shows cell morphology of iPS cells induced with OSMK; SOX(-)(OKM) + hsa-miR-302-367 cluster miRNA (61N2); and OCT3/4 + KLF4(OK) + hsa-miR-302-367 cluster miRNA(13301) (top panel). Fig. 8 also shows that iPS cells induced with OK + hsa-miR-302-367 cluster miRNA (13301) is positive for alkaline phosphatase (red-stained) (bottom panel).

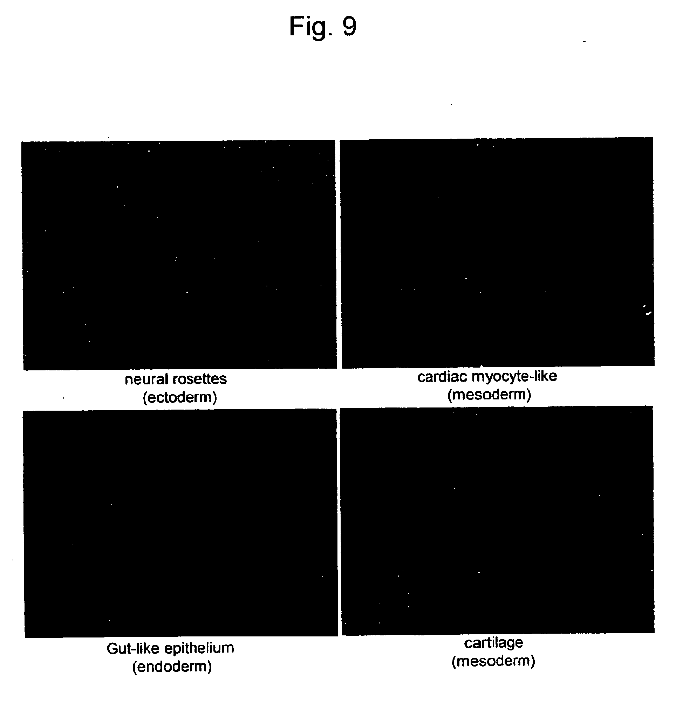

FIG. 9 shows formation of teratoma in a SCID mouse inoculated with human iPS cells, which cells were obtained by introducing OCT3/4 gene, KLF4 gene, c-MYC gene, and hsa-miR-302-367 cluster into human adult fibroblast cells (aHDF). Specifically, into the aHDF-Slc7a1 cells, which are capable of expressing the mouse ecotropic receptor Slc7a1 by transduction via rentivirus, were introduced the genes: OCT3/4, KLF4, and c-MYC, and hsa-miR-302-367 cluster with the aid of retrovirus, thereby generating human iPS cells, which were subsequently used to inoculate them to a mouse. Following the inoculation, the formed teratoma was removed and sectioned, then stained with hematoxylin and eosin for observation of the stained cells.

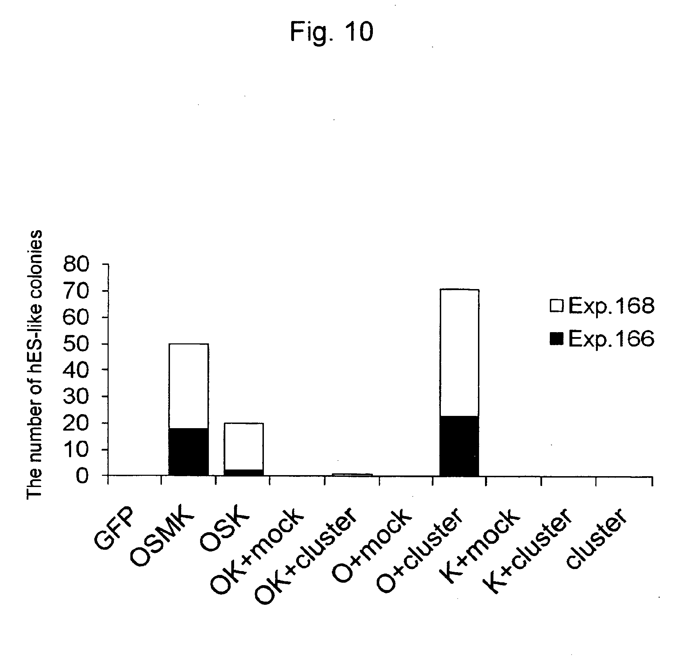

Fig. 10 shows the results obtained when human adult dermal fibroblast cells (aHDF-Slc7a1) were subjected to nuclear reprogramming with a combination of the genes: OCT3/4, KLF4, or OCT3/4 + KLF4, and hsa-miR-302-367 cluster miRNA.

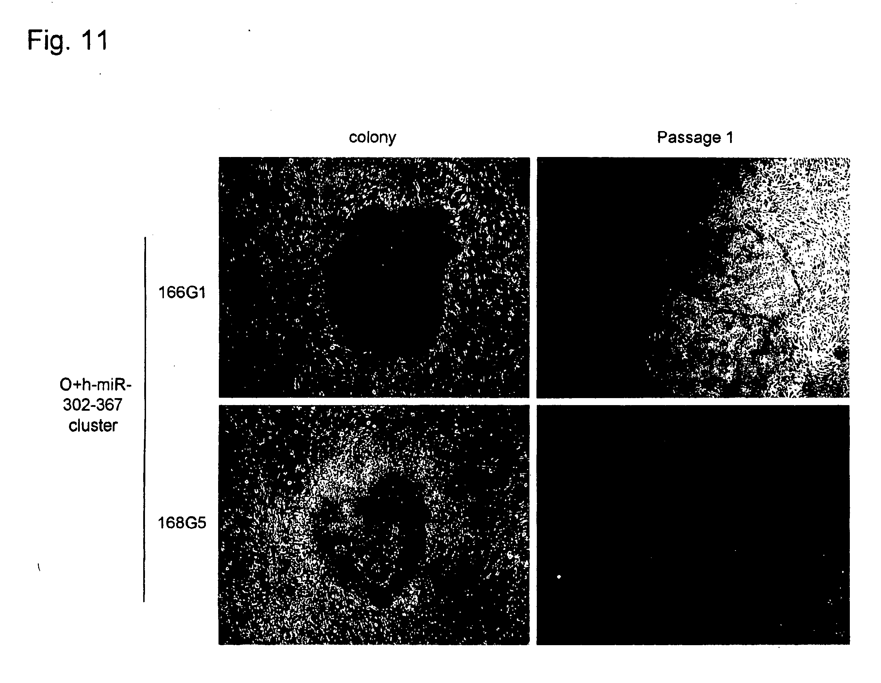

Fig. 11 shows colony images at the time of colony formation and at the first passage of the iPS cells, i.e. 166G1 and 168G5, which were established by introduction of OCT3/4 gene + hsa-miR-302-367 cluster miRNA.

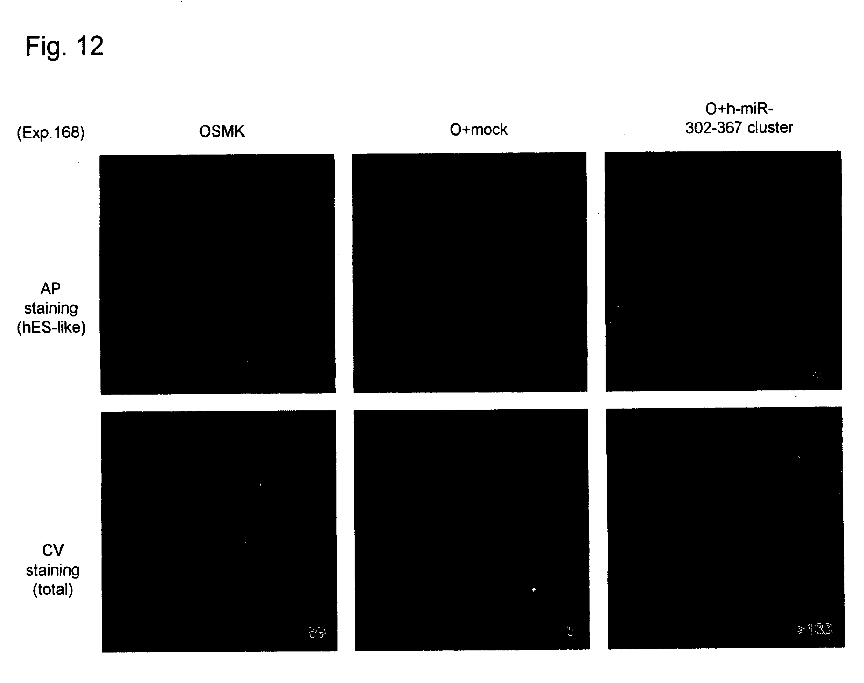

Fig. 12 shows that iPS cells (Exp. 168) established by introduction of OCT3/4 gene + hsa-miR-302-367 cluster miRNA, are positive for alkaline phosphatise (AP) (top panel) and also shows the results of Crystal Violet (CV) staining (bottom panel). This figure further shows the respective results of AP staining and CV staining for iPS cells, which were induced with OSMK or O + mock in Exp. 168 of Example 10, as a positive control or a negative control, respectively.

### BEST MODES FOR CARRYING OUT THE INVENTION

The terms used herein include the following meanings. Other terms are intended to be defined as generally used in the art.

The term "induced pluripotent stem cell" or "iPS cell" as used herein refers to an artificially prepared cell having an ability of differentiation pluripotency, which cell is not a so-called embryonic stem (ES) cell but has properties similar to those of ES cells. These cells were first established from mouse somatic cells by Takahashi and Yamanaka (Cell 126: 663-676, 2006), and it was demonstrated later that such cells could also be established from human somatic cells (WO 2007/069666; Takahashi, K. et al., Cell 131: 861-872, 2007; Yu, J. et al., Science 318: 1917-1920, 2007; Nakagawa, M. et al., Nat. Biotechnol., 26: 101-106, 2008). iPS cells have the capacity of differentiating into various cells constituting animal bodies (i.e., differentiation pluripotency) and the capacity of proliferating semipermanently while maintaining the karyotype, and iPS cells express a group of genes that are generally expressed in ES cells, similarly. Because of such properties, iPS cells are artificially induced by reprogramming somatic cells (i.e., reprogramming), and properties of iPS cells are apparently different from those of differentiated somatic cells.

The term "reprogramming" or "nuclear reprogramming" as used herein also refers to reprogramming, and this term indicates a process and a means in which differentiated cells are induced to be converted into undifferentiated cells and into pluripotent cells, in particular. The phenomenon of reprogramming had been originally observed by methods such as the injection of a somatic cellular nucleus into an ennucleated unfertilized egg and the fusion of an ES cell with a somatic cell. According to the reprogramming technique of the present invention, reprogramming of somatic cells into iPS cells can be realized without depending on germ-line cells, such as egg cells, oocytes, and ES cells.

A factor that enables such reprogramming is a reprogramming factor or a nuclear reprogramming factor. The reprogramming factor used herein includes a protein(s), a nucleic acid(s), or a combination(s) thereof. Examples of known reprogramming factors include combinations of reprogramming factors selected from among the reprogramming factor families, such as the Oct family, the Sox family, the Klf family, the Myc family, and the Lin family, including Oct3/4, Sox2, Klf4, c-Myc, and Lin28.

As used herein, the gene names of reprogramming factors are represented with the use of the same gene names, such as the symbols Oct, Nanog, Sox, Klf, Myc, and Lin, regardless of a mammalian animal type, unless otherwise specified. Thus, although the gene names of Oct, Nanog, Sox, Klf, Myc, and Lin are generally used as mouse gene names, they are used in the description, for humans or other mammalian animals as well as for mice, unless otherwise specified.

Oct3/4 may be also referred to as Oct3, Oct4, or POUSF1, and all of them means the same transcription factor. In the present description, these are collectively referred to as "Oct3/4."

The term "DNA" used herein refers to a deoxyribonucleic acid, such as genomic DNA, gene, or cDNA.

The term "somatic cell" used herein refers to any mammalian animal cells excluding germ-line cells such as egg cells and oocytes, totipotent cells, or ES cells.

The term "mammalian animal" used herein refers to all mammalian animals. Examples of particularly preferable mammalian animals include primates, rodents, ungulates, and pet animals.

The present invention is described in greater detail.

The method for preparing an induced pluripotent stem cell(s) of the present invention comprises a step of nuclear reprogramming with an exogenous nuclear reprogramming factor(s) in the presence of an exogenous miRNA(s), wherein the miRNA(s) has a capacity to offer a higher nuclear reprogramming efficiency in the presence of the miRNA(s) than in the absence of the miRNA(s).

Another method of the present invention is a method for increasing (or enhancing) a nuclear reprogramming efficiency for inducing an induced pluripotent stem cell from a somatic cell, which method comprises reprogramming the somatic cell with an exogenous nuclear reprogramming factor(s) in the presence of at least one exogenous miRNA, so that the number of the resulting induced pluripotent stem cells is more increased than that in the absence of the miRNA, wherein the miRNA has a capacity to offer a higher nuclear reprogramming efficiency in the presence of the miRNA than in the absence of the miRNA.

Thus, the above-described two methods are **characterized in that** the miRNA is the one: (a) that is expressed at a higher level in embryonic stem cells than in somatic cells; and (b) that has a capacity to offer a higher nuclear reprogramming efficiency in the presence of the miRNA than in the absence of the miRNA.

Regarding miRNA, the classification thereof, in vivo functions thereof, and the like are described in, for example, Jikken Igaku (Experimental Medicine), 24, pp. 814-819, 2006; microRNA Jikken Purotokoru (microRNA Experimental Protocols), pp. 20-35, 2008, Yodosha Co., Ltd., Japan, etc.. The nucleotide number of miRNA is, for example, 18 to 25, preferably 20 to 25, and more preferably 21 to 23. At present, a database storing data relating to about 1,000 miRNAs is available (for example, miRBase, http://microrna.sanger.ac.uk/sequences/ index. shtml), and it is possible for those skilled in the art to obtain any miRNA data therefrom and to readily extract miRNA expressed in embryonic stem cells at a higher level than in somatic cells. In addition, it is also possible to readily identify miRNA expressed in embryonic stem cells at a higher level than in somatic cells by confirming differences in miRNA expression between embryonic stem cell and somatic cell with the use of available techniques for those skilled in the art such as miRNA microarray and real-time PCR analyses.

Regarding differences in nuclear reprogramming efficiency in the presence and absence of miRNA, as described specifically in Examples later, a transgenic mouse (i.e., Nanog-IRES-Puro mouse) is generated by insertion of sequences encoding Enhanced Green Fluorescent Protein (EGFP) and a puromycin resistance gene downstream of a Nanog gene promoter region, the expression of which is specific to ES cells, and into the fibroblast cells from the mouse are introduced a nuclear reprogramming factor(s) or a vector(s) comprising DNA encoding a nuclear reprogramming factor(s) and respective miRNAs or a vector(s) comprising DNA encoding the miRNA(s) or a precursor RNA(s) thereof so as to induce nuclear reprogramming, and then the efficiency of induced pluripotent stem cell production is confirmed. Production efficiency can be evaluated by, for example, counting the number of GFP positive colonies. More specifically, the number of colonies may be compared by the following way: drug selection is started from about the 21^{st} day after introduction of the above vectors and miRNA; and the number of total colonies and the number of Nanog GFP positive colonies (GFP, the expression of which is induced by the Nanog promoter, is observable under fluorescent microscopy) are counted on about the 28th day. It should be understood that the confirmation of the nuclear reprogramming efficiency is not limited to the above method, appropriate modification and alteration can be made in the above method, and any appropriate method can be employed by those skilled in the art.

As miRNA, it is preferable to use miRNA derived from the same animal species as the target animal whose somatic cells are to be reprogrammed. Usable miRNA includes wild type miRNAs as well as miRNAs in which several nucleotides (for example 1 to 6 nucleotides, preferably 1 to 4 nucleotides, more preferably 1 to 3 nucleotides, yet more preferably 1 or 2 nucleotides, and particularly preferably 1 nucleotide) are substituted, added, inserted, and/or deleted from the nucleotide sequence of wild-type miRNA, and which are capable of exerting equivalent functions to those of the wild type miRNA in vivo.

Examples of miRNA include one or more miRNAs contained in the following RNA sequences registered in the miRBase: hsa-miR-372 (MI0000780), hsa-miR-373 (MI0000781), hsa-miR-302b (MI0000772), hsa-miR-302c (MI0000773), hsa-miR-302a (MI0000738), hsa-miR-302d (MI0000774), hsa-miR-367 (MI0000775), hsa-miR-520c (MI0003158), mmu-miR-291a (MI0000389), mmu-miR-294 (MI0000392), and mmu-miR-295 (MI0000393). (Numbers in the brackets respectively indicate miRBase accession numbers. The symbol "hsa-miR-" represents human miRNA, and the symbol "mmu-miR-" represents mouse miRNA.)

In the method of the present invention, miRNAs that have been confirmed to improve the nuclear reprogramming efficiency in the above manner can be used either alone or in combination of two or more types. In addition, a plurality of miRNAs forming a cluster may be used. For example, hsa-miR-302-367 may be used as an RNA sequence comprising a miRNA cluster. Examples of RNA sequences to utilize such miRNAs are shown in SEQ ID NOS: 1 to 14 in the Sequence Listing. SEQ ID NO: 1: mmu-miR-294 (MI0000392); SEQ ID NO: 2: mmu-miR-295 (MI0000393); SEQ ID NO: 3: hsa-miR-372 (MI0000780); SEQ ID NO: 4: hsa-miR-373 (MI0000781); SEQ ID NO: 5: hsa-miR-302b (MI0000772); SEQ ID NO: 6: hsa-miR-302c (MI0000773); SEQ ID NO: 7: hsa-miR-302a (MI0000738); SEQ ID NO: 8: hsa-miR-302d (MI0000774); SEQ ID NO: 9: hsa-miR-367 (MI0000775); SEQ ID NO: 10: hsa-miR-520c (MI0003158); SEQ ID NO: 11: mmu-miR-291a MI0000389; SEQ ID NO: 12: hsa-miR-302b-367 cluster; SEQ ID NO: 13: mmu-miR-290 (MI0000388); and SEQ ID NO: 14: hsa-miR-371-373 cluster. Among these RNA sequences, some RNA sequences may contain a plurality of miRNAs within one sequence. Use of such an RNA sequence enables efficient production of iPS cells. Further, an RNA sequence containing a plurality of miRNAs within one sequence and one or more other RNA sequences including one or more miRNAs can also be used in combination. In the present invention, preferably, the miRNAs are one or more miRNAs contained in one or more RNA sequences selected from among SEQ ID NOS: 1 to 5, 10 to 12, and 14 in the Sequence Listing.

miRNA is non-coding RNA which is not translated into a protein. miRNA is first transcribed as pri-miRNA from a corresponding gene, this pri-miRNA then generates pre-miRNA having a characteristic hairpin structure of about 60 to about 120 nucleotides or more, and this pre-miRNA is further processed into mature miRNA, which is mediated by Dicer. In the present invention, not only mature miRNA but also precursor RNA thereof (i.e., pri-miRNA or pre-miRNA), or DNA encoding the miRNA or precursor RNA (e.g., a vector), can be used, as long as the effect of the present invention is not impaired. In addition, miRNA for use in the present invention may be either natural type RNA or non-natural type RNA.

The production method of miRNA for use in the present invention is not specifically limited. For example, a chemical synthetic method or a method using genetic recombination techniques may be employed. When the production is carried out by a method using genetic recombination techniques, miRNA for use in the present invention can be produced through transcription reaction with the use of a DNA template and a RNA polymerase obtained by means of gene recombination. Examples of usable RNA polymerase include a T7 RNA polymerase, a T3 RNA polymerase, and a SP6 RNA polymerase.

Alternatively, a recombinant vector capable of expressing miRNA can be produced by insertion of miRNA-encoding DNA or precursor RNA (pri-miRNA or pre-miRNA)-encoding DNA into an appropriate vector, so that the DNA is under the regulation of expression control sequences (e.g., promoter and enhancer sequences). The type of vector used herein is not specifically limited, and DNA vectors are preferable. Examples thereof include virus vectors and plasmid vectors. The usable virus vectors include, but are not limited to, retrovirus vectors, lentivirus vectors, adenovirus vectors, adeno-associated virus vectors, and the liked. As the above plasmids, mammalian expression plasmids well-known to those skilled in the art can be employed. In the nuclear reprogramming, means to allow a miRNA(s) to exist in cells are not restricted. Examples of such means include, but are not limited to, a method for directly injecting miRNA into somatic cells or a method for introducing a recombinant vector capable of expressing miRNA into somatic cells.

The method for introducing a miRNA(s) and a recombinant vector(s) into somatic cells is not specifically limited, and any method known in the art can be employed. Examples of the methods that can be employed include transfection, microinjection, calcium phosphate precipitation, liposome-mediated transfection, DEAE dextran-mediated transfection, and electroporation.

As a means for confirming a nuclear reprogramming factor, for example, the screening method of nuclear reprogramming factor described in WO 2005/80598 can be employed. Those skilled in the art are able to screen for a nuclear reprogramming factor for use in the method of the present invention by referring to the above publication. In addition, the nuclear reprogramming factor can also be confirmed by using a method in which appropriate modification or alteration has been made in the above screening method.

Examples of the combination of DNAs encoding reprogramming factors (or reprogramming factor genes) are disclosed in WO 2007/69666. In addition, other examples of the combinations of reprogramming factor genes are disclosed in, for example, Science, 318, pp. 1917-1920, 2007, Cell Stem Cell, 3, pp. 568-574, 2008. Accordingly, those skilled in the art are able to understand a diversity of nuclear reprogramming factor gene combinations, and are able to employ appropriate reprogramming factor gene combinations in the method of the present invention, which combinations are not disclosed in WO 2007/069666, Science 318: pp. 1917-1920, 2007, and Cell Stem Cell, 3, pp. 568-574, 2008, by using the screening methods of nuclear reprogramming factor described in WO 2005/80598.

Examples of reprogramming factors and DNAs encoding the reprogramming factors, include one or more reprogramming factors selected from the group consisting of Oct family members, Klf family members, Sox family members, Myc family members, Lin family members, and Nanog, and DNAs encoding the reprogramming factors. In the method of the present invnetion, the nuclear reprogramming factor comprises: (i) no Sox family member and at least (a) Oct 3/4, (b) Oct 3/4 and a Klf family member selected from Klf1, Klf2, Klf4 and Klf5, (c) Oct 3/4 and Nanog, or (d) Oct 3/4 , a Klf family member selected from Klf1, Klf2, Klf4 and Klf5, and an Myc family member selected from c-Myc, N-Myc and L-Myc, or (ii) Oct 3/4 , Sox2 and a Klf family member selected from Klf1, Klf2, Klf4 and Klf5.

Specific examples of said family genes are disclosed in WO 2007/69666 regarding Oct family members, Klf family members, Sox family members, Myc family members, or DNAs encoding the respective members. Those skilled in the art can also extract DNAs encoding Lin family members for the Lin family genes. Examples of such family members are as follows: the Oct family member is Oct3/4, the Klf family member is Klf4, the Myc family member is c-Myc or L-Myc, the Sox family member is Sox2, and the Lin family member is Lin28.

Examples of combinations of DNAs encoding reprogramming factors include:
(a) a combination of DNAs encoding the two reprogramming factors: an Oct family member and an Sox family member;
(b) a combination of DNAs encoding the three reprogramming factors: an Oct family member, a Klf family member, and an Sox family member;
(c) a combination of DNAs encoding the four reprogramming factors: an Oct family member, an Sox family member, the Lin family member, and Nanog;
(d) a combination of DNAs encoding the four reprogramming factors: an Oct family member, a Klf family member, an Sox family member, and an Myc family member;
(e) a DNA encoding the reprogramming factor: an Oct family member;
(f) a combination of DNAs encoding the two reprogramming factors: an Oct family member and a Klf family member;
(g) a combination of DNAs encoding the two reprogramming factors: an Oct family member and Nanog; and
(h) a combination of DNAs encoding the three reprogramming factors: an Oct family member, a Klf family member, and an Myc family member.

The Sox family member may or may not be comprised as a reprogramming factor, preferably the Sox family member (e.g., Sox2) is not comprised.

In the present invention, examples of particularly preferable combinations of DNAs encoding reprogramming factors include:
(1) a DNA encoding the reprogramming factor: Oct3/4;
(2) a combination of DNAs encoding the two reprogramming factors: Oct3/4 and Klf4;
(3) a combination of DNAs encoding the two reprogramming factors: Oct3/4 and Nanog;
(4) a combination of DNAs encoding the three reprogramming factors: Oct3/4, Klf4, and c-Myc; and
(5) a combination of DNAs encoding the three reprogramming factors: Oct 3/4, Klf4 and Sox2.

All of these DNAs are genes that are commonly present in animals, particularly mammals including humans. In order to use the above DNAs in the present invention, genes or cDNAs derived from any mammals (for example, primates, such as humans and monkeys, rodents, such as mice and rats, and ungulates, such as cattle, sheep, pigs, and horses) can be employed. In addition, it is also possible to use wild type gene products, as well as mutant gene products in which several amino acids (for example, 1 to 10 amino acids, preferably 1 to 6 amino acids, more preferably 1 to 4 amino acids, yet more preferably 1 to 3 amino acids, and particularly preferably 1 or 2 amino acids) have been substituted, inserted, and/or deleted, and which have functions equivalent to those of the wild type gene products. As the c-Myc gene product, for example, a stable variant (e.g., T58A) may also be used as well as the wild type. The same applies to other gene products.

In addition to the above-described DNAs, DNAs encoding factors which can induce immortalization of cells may further be combined. As disclosed in WO 2007/069666, for example, one or more genes (or cDNAs) selected from the group consisting of TERT, SV40 Large T antigen, HPV16 E6, HPV16 E7, and Bmil can be used either alone or in combination optionally.

In addition to the above DNAs or genes, one or more genes (or cDNAs) selected from the group consisting of Fbx15, ERas, ECAT15-2, Tcl1, and β-catenin may be combined, and/or one or more genes (or cDNAs) selected from the group consisting of ECAT1, Esg1, Dnmt3L, ECAT8, Gdf3, Myb12, ECAT15-1, Fthl17, Sall4, Rex1, UTF1, Stella, Stat3, and Grb2 may also be combined. These combinations are specifically described in WO 2007/069666.

GenBank accession numbers of sequences (from humans, mice, or rats), such as the above reprogramming factors, for example, Oct3/4, Nanog, Lin28, Lin28b, ECAT1, ECAT2 (also referred to as ESG1), ECAT3 (also referred to as Fbx15), ECAT5 (also referred to as Eras), ECAT7, ECAT8, ECAT9 (also referred to as Gdf3), ECAT10 (also referred to as Sox15), ECAT15-1 (also referred to as Dppa4), ECAT15-2 (also referred to as Dppa2), Fthl17, Sall4, Rex1 (also referred to as Zfp42), Utf1, Tcl1, Stella (also referred to as Dppa3), β-catenin (also referred to as Ctnnb1), Stat3, Grb2, c-Myc, Sox1, Sox2, Sox3, Sox4, Soxl1, Myb12, N-Myc, L-Myc, Klf1, Klf2, Klf4, Klf5 (as described in WO 2007/069666), FoxD3, ZNF206, Mybl2, and Otx2 (as described in WO 2008/118820) are set forth below. The amino acid and nucleotide sequences of such factors are available from GenBank (NCBI, U.S.A.).

Regarding Oct3/4, for example, the sequences of human Oct3/4, mouse Oct3/4, and rat Oct3/4 are registered under the accession numbers NM_203289 or NM_002701, NM_013633, and NM_001009178, respectively.

Regarding Nanog, for example, the sequences of human Nanog, mouse Nanog, and rat Nanog are registered under the accession numbers NM_024865, NM_028016, and NM_001100781, respectively.

Regarding Lin28, for example, the sequences of human Lin28, mouse Lin28, and rat Lin28 are registered under the accession numbers NM_024674, NM_145833, and NM_001109269, respectively.

As a factor that is similar to Lin28, Lin28b that belongs to the same Lin family is known. Regarding Lin28b, for example, the sequences of human Lin28b and mouse

Lin28b are registered under the accession numbers NM_001004317 and NM_001031772, respectively.

Regarding ECAT1, the sequences of human ECAT1 and mouse ECAT1 are registered under the accession numbers AB211062 and AB211060, respectively.

Regarding ECAT2, the sequences of human ECAT2 and mouse ECAT2 are registered under the accession numbers NM_001025290 and NM_025274, respectively.

Regarding ECAT3, the sequences of human ECAT3 and mouse ECAT3 are registered under the accession numbers NM_152676 and NM_015798, respectively.

Regarding ECAT5, the sequences of human ECAT5 and mouse ECAT5 are registered under the accession numbers NM_181532 and NM_181548, respectively.

Regarding ECAT7, the sequences of human ECAT7 and mouse ECAT7 are registered under the accession numbers NM_013369 and NM_019448, respectively.

Regarding ECAT8, the sequences of human ECAT8 and mouse ECAT8 are registered under the accession numbers AB211063 and AB211061, respectively.

Regarding ECAT9, the sequences of human ECAT9 and mouse ECAT9 are registered under the accession numbers NM_020634 and NM_008108, respectively.

Regarding ECAT10, the sequences of human ECAT10 and mouse ECAT 10 are registered under the accession numbers NM_006942 and NM_009235, respectively.

Regarding ECAT15-1, the sequences of human ECAT15-1 and mouse ECAT15-1 are registered under the accession numbers NM_018189 and NM_028610, respectively.

Regarding ECAT15-2, the sequences of human ECAT15-2 and mouse ECAT15-2 are registered under the accession numbers NM_138815 and NM_028615, respectively.

Regarding Fthl17, the sequences of human Fthl17 and mouse Fthl17 are registered under the accession numbers NM_031894 and NM_031261, respectively.

Regarding Sal14, the sequences of human Sal14 and mouse Sal14 are registered under the accession numbers NM_020436 and NM_175303, respectively.

Regarding Rex1, the sequences of human Rex1 and mouse Rex1 are registered under the accession numbers NM_174900 and NM_009556, respectively.

Regarding Utf1, the sequences of human Utf1 and mouse Utf1 are registered under the accession numbers NM_003577 and NM_009482, respectively.

Regarding Tel1, the sequences of human Tcl1 and mouse Tcl1 are registered under the accession numbers NM_021966 and NM_009337, respectively.

Regarding Stella, the sequences of human Stella, mouse Stella are registered under the accession numbers NM_199286 and NM_139218, respectively.

Regarding β-catenin, the sequences of human β-catenin and mouse β-catenin are registered under the accession numbers NM_001904 and NM_007614, respectively.

Regarding Stat3, the sequences of human Stat3 and mouse Stat3 are registered under the accession numbers NM_139276 and NM_213659, respectively.

Regarding Grb2, the sequences of human Grb2 and mouse Grb2 are registered under the accession numbers NM_002086, NM_008163, respectively.

Regarding FoxD3, the sequences of human FoxD3 and mouse FoxD3 are registered under the accession numbers NM_012183 and NM_010425, respectively.

Regarding ZNF206, the sequences of human ZNF206 and mouse ZNF206 are registered under the accession numbers NM_032805 and NM_001033425, respectively.

Regarding Mybl2, the sequences of human Mybl2 and mouse Mybl2 are registered under the accession numbers NM_002466 and NM_008652, respectively.

Regarding Otx2, the sequences of human Otx2 and mouse Otx2 are registered under the accession numbers NM_172337 and NM_144841, respectively.

Regarding c-Myc, the sequences of human c-Myc and mouse c-Myc are registered under the accession numbers NM_002467 and NM_010849, respectively.

Regarding N-Myc, the sequences of human N-Myc and mouse N-Myc are registered under the accession numbers NM_005378 and NM_008709, respectively.

Regarding L-Myc, the sequences of human L-Myc and the sequences of mouse L-Myc are registered under the accession numbers NM_001033081 and NM_008506, respectively.

Regarding Sox1, the sequences of human Sox1 and mouse Sox1 are registered under the accession numbers NM_005986 and NM_009233, respectively.

Regarding Sox2, the sequences of human Sox2 and mouse Sox2 are registered under the accession numbers NM_003106 and NM_011443, respectively.

Regarding Sox3, the sequences of human Sox3 and mouse Sox3 are registered under the accession numbers NM_005634 and NM_009237, respectively.

Regarding Sox4, the sequences of human Sox4 and mouse Sox4 are registered under the accession numbers NM_003107 and NM_009238, respectively.

Regarding Sox11, the sequences of human Sox11 and mouse Sox11 are registered under the accession numbers NM_003108 and NM_009234, respectively.

Regarding Mybl2, the sequences of human Mybl2 and mouse Mybl2 are registered under the accession numbers NM_002466 and NM_008652, respectively.

Regarding Klf1, the sequences of human Klf1 and mouse Klf1 are registered under the accession numbers NM_006563 and NM_010635, respectively.

Regarding Klf2, the sequences of human Klf2 and mouse Klf2 are registered under the accession numbers NM_016270 and NM_008452, respectively.

Regarding Klf4, the sequences of human Klf4, mouse Klf4 are registered under the accession numbers NM_004235 and NM_010637, respectively.

Regarding Klf5, the sequences of human Klf5 and mouse Klf5 are registered under the accession numbers NM_001730 and NM_009769, respectively.

According to the method of the present invention, the reprogramming factors can be employed either in the form of a protein (or a polypeptide) or in the form of a nucleic acid (or a polynucleotide). Such proteins or nucleic acids can be prepared by means of conventional techniques, such as polymerase chain reaction (PCR) techniques, gene engineering techniques, and hybridization techniques. On the basis of known sequences of nucleic acids from given animal species, also, analogous nucleic acids of other animals can be isolated from tissue or genomic libraries. General techniques for doing so are described in Sambrook, J. et al., Molecular Cloning: A Laboratory Manual, 2nd edition, Cold Spring Harbor Laboratory Press, 1989, and Ausubel, F. M. et al., Short protocols in Molecular Biology: A Compendium Methods from Current Protocols in Molecular Biology, John Wiley & Sons, 1999, for example.

The nuclear reprogramming factors of the present invention can include other gene products, which can function as nuclear reprogramming factors, one or more factors involved in differentiation, development, or proliferation, and factors having other physiological activities. It should be understood that the aforementioned embodiments may also fall within the scope of the present invention.

In order to further enhance the efficiency for establishing induced pluripotent stem cells, for example, cytokines, such as a basic fibroblast growth factor (bFGF) and a stem cell factor (SCF), may be added to medium, in addition to the aforementioned miRNA(s). Alternatively, low-molecular-weight compounds, such as histone deacetylase inhibitors (e.g., valpronic acid), DNA metylase inhibitors (e.g., 5'-azacytidine), or histone methyltransferase (G9a) inhibitors (e.g., BIX01294 (BIX)) may be introduced into cells (D. Huangfu et al., Nat. Biotechnol., 26, pp. 795-797, 2008; S. Kubicek et al., Mol. Cell, 25, pp. 473-481, 2007; Y. Shi et al., Cell Stem Cell, 3, 568-574, 2008, Yan Shi et al., Cell Stem Cell, 2, pp. 525-528, 2008). In addition, p53 inhibitors such as shRNA or siRNA against p53 or UTF1 may be introduced into cells (Yang Zhao et al., Cell Stem Cell, 3, pp. 475-479, 2008). Also, activation of the Wnt signal (Marson A. et al., Cell Stem Cell, 3, pp. 132-135, 2008) or inhibition of signaling by TGFβ receptors, mitogen-activated protein kinase, or glycogen synthase kinase-3 (Silva J. et al., PloS Biology, 6, pp. 2237-2247, 2008) can enhance the efficiency of generating iPS cells.

If one or more gene products among the above nuclear reprogramming factor genes are already expressed in somatic cells to be reprogrammed, such gene products can be excluded from the factors to be introduced. For example, one or more DNAs besides the already-expressed one or more genes can be introduced into somatic cells by appropriate gene introduction methods, such as a method involving the use of a vector. Examples of vectors include, but are not limited to, virus vectors, plasmids, and artificial chromosomes. Examples of the virus vectors include retrovirus vectors, lentivirus vectors, adenovirus vectors, adeno-associated vectors, and Sendai virus vectors. Examples of the artificial chromosome vectors include human artificial chromosome (HAC), yeast artificial chromosome (YAC), and bacteria artificial chromosome (BAC and PAC), and HAC and YAC are mini-chromosomes comprising a centromere, two telomeres, and chromosome fragments. Examples of the plasmids that can be used are plasmids for mammalian cells. When two or more DNAs encoding the nuclear reprogramming factors are to be introduced into somatic cells, a cassette can be constructed by ligating such plurality of DNAs in tandem, and control sequences, such as promoter, enhancer, ribosome binder, terminator, or polyadenylation site, may be ligated to each DNA, so as to express DNA, and the resulting cassette may then be inserted into a vector. Alternatively, the respective DNAs may be separately and expressibly inserted into different vectors. The vectors may further comprise a selection marker sequence such as a drug resistance gene (e.g., kanamycin resistance gene, ampicillin resistance gene, or puromycin resistance gene), a selection marker sequence, such as thymidine kinase gene and diphtheria toxin gene, and a reporter gene sequence such as green fluorescent protein (GFP), β-gluclonidase (GUS), or FLAG.

In addition, a nuclear reprogramming factor may be either a protein itself produced from DNA encoding the abovementioned reprogramming factor or in the form of a fusion protein of such a protein with another polypeptide or peptide. Such polypeptide or peptide includes, for example, a histidine tag to facilitate purification of the reprogramming factor protein, and a HIV virus-derived TAT peptide or polyarginine to accelerate endocytosis of a nuclear reprogramming factor through a cell membrane.

As used herein, the term "induced pluripotent stem cell" or "iPS cell" refers to a cell having properties similar to those of ES cell as defined above. Specifically, this term includes undifferentiated cells, which are reprogrammed from somatic cells and have pluripotency and proliferation potencies. This term is not to be construed as limiting in any sense, and it should be construed to have its broadest sense. The preparation methods of induced pluripotent stem cells with the use of nuclear reprogramming factors are described in WO 2005/80598 (the term "ES-like cell" is used in this document), and means for isolating induced pluripotent stem cells are also specifically described therein. In addition, specific examples of the reprogramming factors and specific examples of the methods of reprogramming somatic cells with the use of such reprogramming factors are disclosed in WO 2007/069666. Accordingly, those skilled in the art will be able to refer to these documents for carrying out the present invention.

Methods for producing induced pluripotent stem cells from somatic cells according to the method of the present invention are not specifically limited, and any method can be employed, as long as the methods enable nuclear reprogramming of somatic cells with a nuclear reprogramming factor(s) in the presence of a miRNA(s) under the conditions that somatic cells and induced pluripotent stem cells can grow. For example, a vector which can express a DNA(s) encoding a nuclear reprogramming factor(s) can be used to introduce the DNA(s) into somatic cells, and at either the same or different timing, a miRNA(s) or a recombinant vector(s) which can express the miRNA(s) or a precursor RNA(s) thereof (i.e., precursor RNA such as pri-miRNA or pre-miRNA) can be introduced into the somatic cells. If such vectors are used, two or more DNAs may be incorporated into a vector so as to enable coexpression of respective DNAs in somatic cells.

When a vector(s) that can express a DNA(s) encoding the nuclear reprogramming factor(s) and/or a miRNA(s) (or a vector that can express the miRNA(s)) are introduced into somatic cells, such vector(s) and miRNA(s) can be introduced into somatic cells that have been cultured on feeder cells or into somatic cells without feeder cells. The vector(s) and miRNA(s) can be introduced into somatic cells via conventional techniques, such as microinjection, liposome, lipofection, electroporation, the calcium phosphate method, or viral infection. The viral infection using a retrovirus or lentivirus vector is described in, for example, Cell, 126: 663-676, 2006; Cell, 131: 861-872, 2007; or Science, 318: 1917-1920, 2007. The use of an adenovirus vector is described in Science, 322: 945-949, 2008. The use of a Sendai virus vector is described in Proc. Jpn. Acad. Ser B Phys. Biol. Sci, 2009; 85 (8); 348-362. A specific means for use of a plasmid is described in, for example, Science, 322: 949-953, 2008.

As to the feeder cells, feeder cells for use in culture of embryonic stem cells may be optionally used. Examples of feeder cells can include primary culture cells of 14 or 15 day-mouse embryonic fibroblasts and STO cells of fibroblast cell line, which cells may be treated with either a drug such as mitomycin C or radiation.

Alternatively, an expression vector(s) comprising a DNA(s) encoding a nuclear reprogramming factor(s) and/or an expression vector(s) comprising a DNA(s) encoding a miRNA(s) or precursor RNA(s) thereof (in this case, a single vector may comprise a DNA(s) encoding a reprogramming factor(s) and a DNA(s) encoding a miRNA(s) or precursor RNA(s) thereof) may be introduced into somatic cells to obtain induced pluripotent stem cells. In such a case, somatic cells into which the DNA(s) encoding a reprogramming factor(s) and a miRNA(s) or precursor RNA(s) thereof has been introduced may be cultured under adequate conditions, resulting in promotion of autonomous reprogramming in somatic cells and induction of induced pluripotent stem cells from somatic cells.

Examples of the culture media to prepare induced pluripotent stem (iPS) cells include, but are not limited to, (1) DMEM, DMEM/F12, or DME medium supplemented with 10% to 15% FBS (the medium may optionally contain a leukemia inhibiting factor (LIF), penicillin/streptomycin, puromycin, L-glutamine, non-essential amino acids, β-mercaptoethanol, or the like) and (2) a medium for culture of ES cells supplemented with bFGF or SCF, such as murine ES cell culture medium (e.g., TX-WES medium, Thromb-X) or primate ES cell culture medium (e.g., primate (human or monkey) ES cell culture medium (ReproCELL)).

The procedure of the culture may comprise bringing somatic cells into contact with a reprogramming factor or DNA encoding the same (preferably, a vector) on a DMEM or DMEM/F12 medium containing 10% FBS in the presence of 5% CO₂ at 37°C, followed by culturing them for about 4-7 days, subsequently reseeding the cells on feeder cells (e.g., STO or SNL cells treated with mitomycin C), and, from about 10 days after contact between somatic cells and reprogramming factors or DNAs encoding the reprogramming factors, culturing the cells on primate ES cell culture medium supplemented with bFGF, thereby obtaining iPS cell-like colonies after about 30-45 days of the contact. This procedure is suitable for induction of primate iPS cells including human iPS cells.

Alternatively, culture may be conducted in DMEM medium containing 10% FBS (which can further contain LIF, penicillin/streptomycin, puromycin, L-glutamine, non-essential amino acids, β-mercaptoethanol, or the like, where needed) on feeder cells (e.g., STO or SNL cells treated with mitomycin C) at 37°C in the presence of 5% CO₂, and iPS-like colonies can then be obtained at least about 25 to 30 days thereafter. This procedure can be employed for induction of rodent iPS cells, such as mouse iPS cells.

During the above-mentioned culture, the medium is exchanged with a fresh medium once a day from 2 days after the initiation of culture. The number of somatic cells used for reprogramming may be ranged from approximately 5 x 10³ cells to approximately 5 x 10⁶ cells per 100 cm² of culture dish, although the number of somatic cells is not limited thereto.

In order to enhance the efficiency for iPS cell induction, culture may be conducted at low oxygen concentrations from 5% to 10%.

The iPS cell-like colonies on the dish are treated with a solution containing trypsin and collagenase IV (i.e., a CTK solution), and the residual colonies are then seeded on the above-mentioned feeder cells, followed by culture in the ES cell culture medium in the same manner. Thus, passages of iPS cells can be conducted.

Various media capable of retaining undifferentiation property and pluripotency ofES cells and various media incapable of retaining these properties are known in the art, and appropriate combination of these media enables efficient isolation of induced pluripotent stem cells. The differentiation ability and proliferation potency of thus isolated induced pluripotent stem cells can be readily checked by those skilled in the art, with the use of general checking means for ES cells. In addition, colonies of induced pluripotent stem cells can be obtained by growing thus produced induced pluripotent stem cells under appropriate conditions, and the presence of these induced pluripotent stem cells can be identified based on the shapes of the colonies. For example, it is known that mouse induced pluripotent stem cells form swollen colonies, while human induced pluripotent stem cells form flat colonies. These colony shapes are respectively very similar to those of mouse ES cell and human ES cell colonies, and those skilled in the art are thus able to identify these produced induced pluripotent stem cells based on the shapes of the colonies.

Induced pluripotent stem (iPS) cells can be identified by, for example, a test based on properties of differentiated pluripotent cells, such as ES cells. When cells are tested in respect of expression of ES cell-specific marker genes, semipermanent cell proliferation capacity, differentiation pluripotency (i.e., formation of three embryonic germ layers), and the like and the tested cells are determined to have such properties, such cells are identified to be iPS cells (Takahashi, K. et al., Cell 131: 861-872, 2007).

Examples of ES cell-specific marker genes include Oct3/4, Nanog, Lin28, PH34, Dnmt3b, Nodal, SSEA3, SSEA4, Tra-1-60, Tra-1-81, and Tra2-49/6F (alkaline phosphatase). By performing RT-PCR involving the use of primers specific for such gene amplification, expression of the above marker genes in cells can be detected.

As to the semipermanent proliferation capacity, whether cells proliferate exponentially is tested by culturing the cells over about 4 to 6 months. In the case of human iPS cell colonies, because the population doubling time is known to be about 46.9 ± 12.4 hours, 47.8 ± 6.6 hours, or 43.2 ± 11.5 hours for example, this value can be used as an indicator of the proliferation capacity (Takahashi, K. et al., Cell 131: 861-872, 2007). Since iPS cells have high telomerase activity, telomerase activity may be detected by, for example, the telomeric repeat amplification protocol (TRAP).

As to the differentiation pluripotency (i.e., formation of three embryonic germ layers), this ability can be confirmed by, for example, forming teratoma and identifying tissues (or cells) of three embryonic germ layers (i.e., ectoderm, mesoderm, and endoderm) in the teratoma tissues. In the case of mouse iPS cells, specifically, cells are injected intradermaly in a nude mouse. In the case of human iPS cells, cells are injected into the spermary of a SCID mouse, followed by confirmation of tumor formation. Further, whether the tumor tissues are composed of tissues, including cartilage tissues (or cells), neural tube tissues (or cells), muscle tissues (or cells), fat tissues (or cells), or gut-like tissues (or cells) is confirmed.

Based on the results of the above tests, iPS cells are identified, and iPS cell colonies can be selected.

The types of somatic cells to be reprogrammed by the method of the present invention are not specifically limited, and any somatic cells can be used. For example, mammalian animals from which somatic cells are derived are not particularly limited, and any animal species may be used. Examples of preferable mammalian animals include primates (e.g., human, monkey, and chimpanzee), rodents (e.g., mouse, rat, hamster, and guinea pig), ungulates (e.g., cattle, sheep, goat, horse, and pig), and pet animals (e.g., dog or cat). Mammalian animals are more preferably humans and mice. Somatic cells may be fetal somatic cells, neonatal somatic cells, or matured somatic cells, without particular limitation. Further, cultured primary cells, subcultured cells, and established cell lines may also be used. Also, tissue stem cells and tissue precursor cells may be used. Specific examples of somatic cells include, but are not limited to: (1) tissue stem cells (somatic stem cells), such as neural stem cells, hematopoietic stem cells, mesenchymal stem cells, and dentis stem cells; (2) tissue precursor cells; and (3) differentiated cells, such as lymphocytes, epidermal cells, endothelial cells, muscle cells, fibroblast cells (e.g., skin fibroblast cells), pilary cells, liver cells, gastric mucosa cells, intestine cells, spleen cells, pancreatic cells (including pancreatic exocrine cells), brain cells, lung cells, renal cells, and skin cells. When induced pluripotent stem cells are used to treat diseases, use of somatic cells isolated from a patient is preferable. For example, somatic cells involved in a disease and somatic cells associated with a therapy for a disease can be used.

Provided are induced pluripotent stem cells induced from somatic cells by the above-described methods. Also provided are somatic cells induced by differentiation of such induced pluripotent stem cells, and a method for inducing the same.

The applications of the induced pluripotent stem cells produced by the methods of the present invention are not specifically limited, and these cells can be used as alternatives for every examination/study to be performed with the use of ES cells and for treatment of diseases using ES cells. The induced pluripotent stem cells can be induced into a variety of differentiated cells, precursor cells, and tissues because of the differentiation pluripotency thereof. For example, the induced pluripotent stem cells obtained by the methods of the present invention may be treated with factors, such as retinoic acid, a growth factor such as EGF, glucocorticoid, activin A/BMP4 (bone morphogenetic protein 4), or VEGF (vascular endothelial growth factor), so that differentiated cells of interest (e.g., nerve cells, myocardial cells, blood cells, and insulin-producing cells) can be induced. Further, techniques for converting the resulting differentiated cells or precursor cells into tissue have also become known. For the purpose of regenerative medicine such as stem cell therapy through autologous cell transplantation by returning the thus-obtained differentiated cells or tissue into a patient, the induced pluripotent stem cells can be utilized.

Further, the induced pluripotent stem (iPS) cells can be introduced into the blastocyst from a mammalian animal (excluding a human), and the blastocyst can be transplanted into the uterus of a surrogate mother of the same animal species. Thus, chimeric animals to which part of the genotypes and traits of the iPS cells has been transmitted can be created (WO 2007/069666). The iPS cells can also be used for modification of a given gene, clarification of gene functions via knock-out or knock-in of a gene, creation of a disease model, or production of a substance (e.g., a protein).

The present invention further provides a kit for inducing an induced pluripotent stem cell from a somatic cell. The kit comprises a combination(s) of components (a) to (d) shown below: i.e., at least one miRNA and at least one nuclear reprogramming factor or at least one vector containing DNA encoding the factor. Such components may be separately placed and packaged in separate containers. The analogous components may be accommodated in the same container. Combinations of components are exemplified as follows:
(a) a combination of a miRNA, or a vector encoding the miRNA, with:
   (i) Oct3/4, or a vector containing DNA encoding Oct3/4; or
   (ii) Oct3/4, or a vector containing DNA encoding Oct3/4, and a Klf family member selected from Klf1, Klf2, Klf4 and Klf5, or a vector containing DNA encoding the Klf family member; or
   (iii) Oct3/4, or a vector containing DNA encoding Oct3/4, and Nanog, or a vector containing DNA encoding Nanog;
   wherein the miRNA is the hsa-miR-302-367 cluster or mature miRNA(s) thereof comprising 18 to 25 nucleotides; or
(b) a combination of a miRNA, or a vector encoding the miRNA, with Oct3/4, or a vector containing DNA encoding Oct3/4, a Klf family member selected from Klf1, Klf2, Klf4 and Klf5, or a vector containing DNA encoding the Klf family member, and an Myc family member selected from c-Myc, N-Myc and L-Myc, or a vector containing DNA encoding the Myc family member, wherein the miRNA is at least one miRNA selected from the group consisting of: mmu-miR-295/295*, the hsa-miR-302-367 cluster and mature miRNAs thereof comprising 18 to 25 nucleotides; or
(c) a combination of a miRNA, or a vector encoding the miRNA, with Oct3/4, or a vector containing DNA encoding Oct3/4, a Klf family member selected from Klf1, Klf2, Klf4 and Klf5, or a vector containing DNA encoding the Klf family member, and Sox2, or a vector containing DNA encoding Sox2, wherein the miRNA is one or more miRNAs selected from the group consisting of:
   - hsa-miR-372;
   - hsa-miR-373 or hsa-miR-373/373*;
   - hsa-miR-371-373 cluster;
   - hsa-miR-302b or hsa-miR-302b/302b*;
   - hsa-miR-302-367 cluster;
   - hsa-miR-520c or hsa-miR-520c-5p/520c-3p;
   - mmu-miR-291 a or mmu-miR-291a-5p/291a-3p;
   - mmu-miR-294 or mmu-miR-294/294*;
   - mmu-miR-295 or mmu-miR-295/295*; and
   - mature miRNAs thereof comprising 18 to 25 nucleotides; or

The miRNA as decribed in any of (a) to (d) above is, for example, one or more mature miRNAs comprising 18 to 25 nucleotides which are cut out from said miRNAs.

Further, preferably, the Klf family member is Klf4, and the Myc family member is c-Myc.

The above-described components may be in the form of either a solution or a solid. A stabilizer for stabilizing the components may be contained. The solid is, for example, a lyophilized solid.

The present invention will be further described in more detail with reference to the following examples, but it should be understood that the scope of the present invention is not limited by these examples.

### EXAMPLES

### Example 1: Preparation of induced pluripotent stem cells via nuclear reprogramming of mouse embryonic fibroblasts

pMXs-based retrovirus vectors, which respectively encode each of three genes of mouse-derived Oct3/4, Sox2, and Klf4, control DsRed, and each miRNA of 18 types of miRNAs, were transfected into PLAT-E cells using FuGENE 6 reagent (Roche). On the next day, embryonic (or fetal) fibroblasts (Nanog GFP MEF, WO 2007/069666) from transgenic mice generated by ligating sequences encoding EGFP and the puromycin resistance gene to a site downstream of a Nanog gene promoter region, were seeded at 1 x 10⁵ cells/well in 6-well plates. On the next day, these cells were reprogrammed with a mixture of a retrovirus expressing three factors Oct3/4, Sox2 and Klf4 and a retrovirus expressing one miRNA selected from 18 miRNAs by infecting the cells with the mixture of retroviruses in a ratio of 1 ml of the three factor expressing retrovirus to 1 ml of the miRNA expressing retrovirus, thereby preparing induced pluripotent stem cells.

**Table 1**

| miRNA number | miRNA sequence (parenthesis: another name) | miRBase accession number |
|---|---|---|
| 1 | mmu-miR-150 | MI0000172 |
| 2 | mmu-miR-182 | MI0000224 |
| 3 | mmu-miR-126 | MI0000153 |
| 4 | mmu-miR-290-295 cluster | |
| 5 | mmu-miR-290 (mmu-miR-290-5p/290-3p) | MI0000388 |
| 6 | mmu-miR-291a (mmu-miR-291a-5p/291a-3p) | MI0000389 |
| 7 | mmu-miR-292 (mmu-miR-292-5p/292-3p) | MI0000390 |
| 8 | mmu-miR-294 (mmu-miR-294/294*) | MI0000392 |
| X(9) | mmu-miR-295 (mmu-miR-295/295*) | MI0000393 |
| 10 | mmu-miR-17-92 cluster | |
| 11 | mmu-miR-323 | MI0000592 |
| 12 | mmu-miR-130b | MI0000408 |
| 13 | mmu-miR-7a-1 | MI0000728 |
| 14 | mmu-miR-7a-2 | MI0000729 |
| 15 | mmu-miR-205 | MI0000248 |
| 16 | mmu-miR-200a | MI0000554 |
| 17 | mmu-miR-200c | MI0000694 |
| 18 | mmu-miR-mix¹⁾ | |

| | | |
|---|---|---|
| * Star form of miRNA. ¹⁾ Introduction of a mixture of the viruses which were prepared separately using retrovirus vectors encoding miRNAs 1 to 17. | | |

From the third day after infection, the cells were cultured in an ES cell medium containing LIF. On the fourth day after infection, the cells were peeled, and the whole amount thereof was reseeded on feeder cells. Every other day thereafter, the ES cell medium containing LIF was exchanged. From the 21^{st} day after infection, drug selection was initiated with the addition of puromycin to a final concentration of 1.5 µg/ml. On the 28^{th} day, the number of Nanog GFP-positive colonies (GFP, the expression of which is induced by a Nanog promoter, can be observed under fluorescent microscope) was counted. As a control, DsRed was used in place of miRNA. The results are shown in Fig. 1. It was found that mmu-miR-294 and mmu-miR-295 could respectively improve the nuclear reprogramming efficiency when introduced into mouse embryonic fibroblasts together with three factors: Oct3/4, Sox2, and Klf4, and could realize efficient establishment of induced pluripotent stem cells.

### Example 2: Preparation of induced pluripotent stem cells via nuclear reprogramming of mouse tail tip fibroblasts

pMXs-based retrovirus vectors, which respectively encode each of the three genes of mouse-derived Oct3/4, Sox2 and Klf4, and either DsRed (as a control) or mmu-miR-295, were transfected into PLAT-E cells using FuGENE 6 reagent (Roche). On the next day, tail tip fibroblasts (Nanog GFP tail tip fibroblasts) from transgenic mice generated by ligating sequences encoding EGFP and the puromycin resistance gene to a site downstream of a Nanog gene promoter region, were seeded at 1 x 10⁵ cells/well in 6-well plates. On the next day, these cells were infected with retroviruses expressing three genes: Oct3/4, Sox2, and Klf4, and either DsRed or mmu-miR-295 at the ratio of 1:1:1:1, so as to prepare induced pluripotent stem cells via nuclear reprogramming.

Since the third day after infection, the cells were cultured in an ES cell medium containing LIF. On the fourth day after infection, the cells were peeled, and the whole amount thereof was reseeded on feeder cells. Every other day thereafter, the ES cell medium containing LIF was exchanged. From the 7^{th}, 21^{st}, or 28^{th} day after infection, drug selection was initiated with the addition of puromycin to a final concentration of 1.5 µg/ml. On the 39^{th} day, the number of total colonies and the number of Nanog GFP positive colonies (GFP, the expression of which is induced by the Nanog promoter, can be observed under fluorescent microscope) were counted. The results are shown in Fig. 2. It was found that mmu-miR-295 could improve the nuclear reprogramming efficiency when introduced into mouse tail tip fibroblasts together with three genes: Oct3/4, Sox2, and Klf4, it could accelerate the reprogramming speed, and it could realize efficient establishment of induced pluripotent stem cells.

### Example 3: Preparation of induced pluripotent stem cells via nuclear reprogramming of adult human dermal fibroblasts

pMXs-based retrovirus vectors, which encode three human-derived genes: OCT3/4, SOX2, and KLF4, control DsRed, and each of 23 miRNAs or miRNA clusters, were transfected into PLAT-E cells using FuGENE 6 reagent (Roche). On the next day, adult human dermal fibroblasts (aHDF-Slc7a1), in which a mouse retrovirus receptor Slc7a1 (aHDF-Slc7a1) was expressed, were seeded at 3 x 10⁵ cells/well in 6-cm dishes. On the next day, the cells were infected with retroviruses expressing three genes: OCT3/4, SOX2, and KLF4, and each type of miRNAs at a ratio of 1:1:1:1, so as to produce induced pluripotent stem cells through nuclear reprogramming.

**Table 2**

| miRNA number | miRNA sequence (parenthesis: another name) | miRBase accession number |
|---|---|---|
| 1 | hsa-miR-371 (hsa-miR-371-5p/371-3p) | MI0000779 |
| 2 | hsa-miR-372 | MI0000780 |
| 3 | hsa-miR-373 (hsa-miR-373/373*) | MI0000781 |
| 4 | hsa-miR-371-373 cluster | |
| 5 | hsa-miR-93 (hsa-miR-93/93*) | MI0000095 |
| 6 | hsa-miR-302a (hsa-miR-302a/302a*) | M I0000738 |
| 7 | hsa-miR-302b (hsa-miR-302b/302b*) | MI0000772 |
| 8 | hsa-miR-302c (hsa-miR-302c/302c*) | MI0000773 |
| 9 | hsa-miR-302d (hsa-miR-302d/302d*) | MI0000774 |
| 10 | hsa-miR-367 (hsa-miR-367/367*) | MI0000775 |
| 11 | hsa-miR-302-367 cluster | |
| 12 | hsa-miR-520a (hsa-miR-520a-5p/520a-3p) | MI0003149 |
| 13 | hsa-miR-520b | MI0003155 |
| 14 | hsa-miR-520c (hsa-miR-520c-5p/520c-3p) | MI0003158 |
| 15 | hsa-miR-520d (hsa-miR-520d-5p/520d-3p) | MI0003164 |
| 16 | hsa-miR-520e | MI0003143 |
| 17 | mmu-miR-290-295 cluster | |
| 18 | mmu-miR-290 (mmu-miR-290-5p/290-3p) | MI0000388 |
| 19 | mmu-miR-291a (mmu-miR-291a-5p/291a-3p) | MI0000389 |
| 20 | mmu-miR-292 (mmu-miR-292-5p/292-3p) | MI0000390 |
| 21 | mmu-miR-293 (mmu-miR-293/293*) | MI0000391 |
| 22 | mmu-miR-294 (mmu-miR-294/294*) | MI0000392 |
| 23 | mmu-miR-295 (mmu-miR-295/295*) | MI0000393 |

On the sixth day after infection, the cells were peeled, and the whole amount of 5 x 10⁵ cells was reseeded on feeder cells. Every other day thereafter, human ES cell medium containing bFGF (ReproCELL) was exchanged. On the 24^{th}, 32^{nd}, and 40^{th} day, the total number of colonies and the number of colonies having morphology of human ES-like cells were counted. As a control, DsRed was used in place of miRNA. The results are shown in Fig. 3. It was found that the number of iPS cell colonies exhibiting ES cell-like morphology increased twice or more, as compared to the control, via introduction of the three genes in the presence of hsa-miR-372, 373, 302b, 302-367 cluster (containing 302b, 302c, 302a, 302d, and 367), 520c, mmu-miR-291a, 294, or 295.

### Example 4: Preparation of induced pluripotent stem cells via nuclear reprogramming of adult human dermal fibroblasts

3 x 10⁵ aHDF-Slc7a1 cells were subjected to plate culture on 60-mm gelatin-coated dishes and infected with retroviruses expressing DsRed, or the four genes (OCT3/4, SOX2, c-MYC, and KLF4), or the three genes (OCT3/4, SOX2, KLF4) in the presence of different miRNAs, independently. Six days after infection, 5 x 10⁵ aHDF-Slc7a1 cells were reseeded on mytomicin-C treated STO cells. Forty days after infection, the number of colonies exhibiting human ES-like morphology was counted. The same experiment was repeated three times.

Fig. 4A shows the results of three independent experiments. It was found that the number of induced pluripotent stem cell colonies increased, as compared to the control, via introduction of genes of the three factors in the presence of hsa-miR-372, 373/373* (hsa-miR-373), 371-373 cluster (containing 371, 372, and 373), 302b/302b* (hsa-miR-302b), 302-367 cluster (containing 302b, 302c, 302a, 302d, and 367), 520c-5p/520c-3p (hsa-miR-520c), mmu-miR-290-5p/290-3p (mmu-miR-290), mmu-miR-291a-5p/291a-3p (mmu-miR-291a), 294/294* (mmu-miR-294), or 295/295* (mmu-miR-295).

Fig. 4B shows the morphology of ES-like colonies of iPS cells observed under microscope.

### Example 5: Expression of ES cell markers in iPS cells produced via nuclear reprogramming of mouse tail tip fibroblasts (TTFs) with the four genes (OCT3/4, SOX2, c-MYC, and KLF4) and with the three genes (OCT3/4, SOX2, and KLF4) + mmu-miR-295/295*

5 x 10⁴ FbNg TTFs (TTFs derived from Fbx15-βgeo/Nanog-IRES-Puro^{r} reporter mouse) cells were subjected to plate culture on gelatin-coated 6-well plates and infected with retroviruses expressing the three genes (Oct3/4, Sox2, Klf4) and either DsRed (Myc(-)3f+DsRed), mmu-miR-295/295* (Myc(-)3f+mmu-miR-295/295*), or c-Myc (four genes). Four days after infection, all the cells (Myc(-)3f + DsRed; Myc(-)3f + mmu-miR-295/295*) or 20-fold diluted cells (4 factors) were reseeded on puromycin and hygromycin-resistant-MSTO (PH-MSTO) cells. Puromycin selection was initiated on days 7, 14, 21, and 28.

RT-PCR analysis using the Rever Tra Ace Kit (Takara) showed that the iPS cells obtained via introduction of the four genes (OCT3/4, SOX2, c-MYC, and KLF4) or the three genes (OCT3/4, SOX2, and KLF4) + mmu-miR-295/295* expressed the ES cell specific marker genes (i.e., Oct3/4, Sox2, and Nanog) and that the expression levels thereof were equivalent to those obtained with the use of mouse ES cells (ES) and mouse iPS cells (Fbx iPS) (Fig. 5).

### Example 6: Preparation of induced pluripotent stem cells via nuclear reprogramming of mouse embryonic fibroblasts using three genes (Oct3/4, Klf4, and c-Myc) and miRNA

1 x 10⁵ Nanog MEF (MEF derived from Nanog-IRES-Puro^{r} reporter mouse) cells were subjected to plate culture on gelatin-coated 6-well plates and infected with retroviruses expressing three genes (Oct3/4, c-MycWT (wild type), and Klf4), and mmu-miR-290-295 cluster, 290-5p/290-3p (mmu-miR-290), 291a-5p/291a-3p (mmu-miR-291), 292-5p/292-3p (mmu-miR-292), 293/293* (mmu-miR-293), 294/294* (mmu-miR-294), or 295/295* (mmu-miR-295) miRNA, at the ratio of 1:1. Four days after infection, half of the cells were reseeded on puromycin and hygromycin C-resistant-MSTO (PH-MSTO) cells. Puromycin selection was initiated 14 days after infection.

Fig. 6A shows the number of Nanog GFP positive colonies. The results of three independent experiments are shown with different colors. "DsRed" indicates the combination of Oct3/4, Klf4, c-Myc, and DsRed.

Fig. 6B shows the results of RT-PCR analysis. RT-PCR analysis using the Rever Tra Ace Kit (Takara) showed that the iPS cells transfected with the four genes (OCT3/4, SOX2, c-MYC, and KLF4) or with the three genes (OCT3/4, c-MycWT, and KLF4) + mmu-miR-290-295 cluster miRNA, 291a-5p/291a-3p, 294/294* or 295/295* expressed the ES cell-specific marker genes (i.e., Oct3/4, Sox2, and Nanog) and that the expression levels thereof were equivalent to those obtained with the use of mouse ES cells (ES) and mouse iPS cells (Fbx iPS).

### Example 7: iPS cell induction with Fb-Ng MEF (MEF derived from Fbx15-βgeo/Nanog-IRES-Puro^{r} reporter mouse) cells expressing Oct3/4, c-Myc, and Klf4 ("Sox(-)") + mmu-miR-295/295* or aHDF-Slc7a1 cells expressing Oct3/4, c-Myc, and Klf4 ("Sox(-)") + hsa-miR-302-367 cluster miRNA

1 x 105 Fb-Ng MEF (MEF derived from Fbx15-βgeo/Nanog-IRES-Puror reporter mouse) cells were subjected to plate culture on gelatin-coated 6-well plates and infected with retroviruses expressing the three genes (Oct3/4, c-MycWT (wild type), Klf4) + miR-295/295*or hsa-miR-302-367 cluster miRNA. Four days after infection, cells were reseeded on puromycin and hygromycin C-treated STO cells (PH-MSTO) in 6-well or 10-cm dishes. Puromycin selection was initiated 7 days after infection.

Fig. 7A shows morphology of iPS cells obtained by introducing Oct3/4, c-Myc, and Klf4 ("Sox(-)") + mmu-miR-295/295*. The colonies were positive for GFP and showed morphology similar to that of ES cells.

Fig. 7B shows a chimera mouse obtained from iPS cells induced with the use of Sox(-)3f + mmu-miR-295/295*.

Fig. 7C shows the results of experiment in which embryoid body (EB)-mediated differentiation was induced *in vitro* from human iPS cells. Human iPS cells (61B1, 61N2), which were established via introduction of the four genes (OCT3/4, KLF4, SOX2, and c-MYC, i.e. "OSMK") or the three genes (OCT3/4, KLF4, and c-MYC, i.e., "OMK(SOX(-))") + hsa-miR-302-367 cluster miRNA, were subjected to plate culture on dishes, and embryoid bodies were formed on 100-mm dishes in accordance with the method described in Takahashi et al., Cell 131: 861-872, 2007. After culturing for 2 weeks, the cells were immunostained with an antibody against each of α-fetoprotein (R&D systems, a differentiation marker for endodermal cells), α-smooth muscle actin (DAKO, a differentiation marker for mesodermal cells), and glial fibrillary astrocytic protein (GFAP) (DAKO, a differentiation marker for ectodermal cells). Expression of each marker was confirmed via staining. Nuclei were stained with Hoechst 33342 (Invitrogen).

### Example 8: iPS cell induction with four genes (OCT3/4, SOX2, MYC, and KLF4), three genes (OCT3/4, MYC, and KLF4, i.e., "SOX(-)3"), or two genes (OCT3/4 and KLF4) in the presence or absence of different miRNAs

3 x 10⁵ aHDF-Slc7a1 cells were subjected to plate culture on 60-mm gelatin-coated dishes, and the four genes (OCT3/4, SOX2, c-MYC, and KLF4; i.e., OSMK), the three genes (OCT3/4, c-MYC, and KLF4; i.e., SOX(-) the three genes (OMK)) in the presence or absence of miRNA (OMK: mock or miRNAs = 2.5:1.5), or the two genes (OCT3/4 + KLF4 (OK)) in the presence or absence of miRNA, were introduced with the aid of a retrovirus vector. DsRed was introduced as a control. Six days after infection, 5 x 10⁵ aHDF-Slc7a1 cells were reseeded on mytomicin C-treated STO cells (MSTO cells). Forty days after infection, the number of ES cell-like colonies was counted. Fig. 8 shows the morphology of observed colonies and the results of analysis confirming that iPS cells obtained via introduction of OK + miRNA were positive for alkaline phosphatase and in undifferentiated state.

Table 3 shows the number of iPS cell colonies exhibiting human ES (hES) cell-like morphology obtained by introducing OSMK, OMK in the presence or absence of miRNA, or OK in the presence or absence of miRNA, into aHDF-Slc7a1 cells. The hES cell-like colonies observed via introduction of OSMK, OMK+miRNAs (hsa-miR-371-373 cluster, hsa-miR-302-367 cluster, or hsa-miR-371-373 cluster +302-367 cluster), or OK + miRNAs (hsa-miR-371-373 cluster, hsa-miR-302-367 cluster, or hsa-miR-371-373 cluster + 302-367 cluster) were detected by six independent experiments (Exp. 54, 61, 63, 114, 130, and 133).

**Table 3**

| Number of hES-like colonies | | | Exp.54 | Exp.61 | Exp.63 | Exp.114 | Exp.130 | Exp.133 |
|---|---|---|---|---|---|---|---|---|
| A | control | DsRed | 0 | 0 | 0 | 0 | 0 | 0 |
| B | OSMK | Y4f(O:S:M:K=1:1:1:1) | 5 | 41 | 54 | 37 | 39 | 100 |
| G | | Y4f(OMK-S=25:15) | | | 13 | 7 | 4 | 22 |
| H | OMK+mock or miRNA | Soxl(-)Y3f+mock | 0 | 0 | 0 | 0 | 0 | 0 |
| I | | Sox(-)Y3f+h-miR-371-373 cluster | 0 | 0 | 1 | 0 | 0 | 0 |
| J | | Sox(-)Y3f+h-miR-302-357 cluster | 0 | 2 | 3 | 0 | 8 | 5 |
| K | | Sox(-)Y3f+h-mir-371-373 cluster+302-367cluster | | | | 0 | 0 | 6 |
| M | OK+mock or miRNA | OK+mock | | | | 0 | 0 | 0 |
| N | | OK+h-miR-371-373 cluster | | | | 0 | 0 | 0 |
| O | | OK+h-miR-302-367 cluster | | | | 0 | 0 | 4 |
| P | | OK+h-miR-371-373 cluster+302-367 cluster | | | | 1 | 0 | 0 |

Fig. 8 shows cell morphology of iPS cells induced with OSMK (61B1); OMK (SOX(-)) + hsa-miR-302-367 cluster miRNA (61N2); and OK + hsa-miR-302-367 cluster miRNA (13301) (top panel). Fig. 8 also shows that iPS cells induced with OK + hsa-miR-302-367 cluster miRNA (13301) are positive for alkaline phosphatase (bottom panel).

Moreover, the present inventors have confirmed that the combination of OCT3/4 and NANOG genes can induce iPS cells (or hES-like cells) from somatic cells.

### Example 9: Confirmation of differentiation pluripotency of iPS cells

Human iPS cells (61N2), which were obtained by introducing OCT3/4, KLF4, c-MYC (i.e., SOX(-)3F), and hsa-miR-302-367 cluster, were intradermally inoculated into the lateral abdomen of the back of a SCID mouse. Nine weeks after inoculation, a tumor was removed, fixed, and embedded in paraffin. The resulting thin sections were stained with hematoxylin and eosin. The histological observation showed that the teratoma contained a variety of tissues including neural tubes (ectoderm), cardiac myocyte-like structure (mesoderm), gut-like epithelium (endoderm), and cartilage (mesoderm). This demonstrates that iPS cells (61N2) have pluripotency, so that iPS cells can differentiate into three embryonic germ layers (Fig. 9).

### Example 10: Induction of iPS cells with a single gene (OCT3/4) in the presence of miRNA

aHDF-Slc71 cells were seeded on a 6-well plate at 1 x 10⁵ cells per well. On the next day, any of the following combinations of genes was introduced into the cells via retrovirus vector.
1. GFP gene (a negative control)
2. OCT3/4 gene, SOX2 gene, c-MYC gene, and KLF4 gene (1:1:1:1)
3. OCT3/4 gene, SOX2 gene, and KLF4 gene (1:1:1)
4. OCT3/4 gene, KLF4 gene, and mock gene (i.e., pMSx empty vector) (1:1:1)
5. OCT3/4 gene, KLF4 gene, and hsa-miR-302-367 cluster (1:1:1)
6. OCT3/4 gene and mock gene (1:1)
7. OCT3/4 gene and hsa-miR-302-367 cluster (1:1)
8. KLF4 gene and mock gene (1:1)
9. KLF4 gene and hsa-miR-302-367 cluster (1:1)
10. hsa-miR-302-367 cluster

Six days after infection, the cells were peeled, and 5 x 10⁵ cells of which were reseeded on MSTO feeder cells. Every other day thereafter, human ES cell medium containing bFGF (ReproCELL) was exchanged, and the number of colonies having ES cell-like morphology was counted 40 days after infection. Results of independent duplicate experiments are shown in Table 4 and in Fig. 10 (Fig. 10 shows graphed results of Table 4).

**Table 4**

| The number of hES-like colonies | Exp.166 | Exp.168 |
|---|---|---|
| GFP | 0 | 0 |
| OSMK | 18 | 32 |
| OSK | 2 | 18 |
| OK+mock | 0 | 0 |
| OK+cluster | 0 | 1 |
| O+mock | 0 | 0 |
| O+cluster | 23 | 48 |
| K+mock | 0 | 0 |
| K+cluster | 0 | 0 |
| cluster | 0 | 0 |

When the OCT3/4 gene was introduced in the presence of hsa-miR-302-367 cluster, iPS cell colonies having ES cell-like morphology were obtained. In this case, the colony counts were greater than those in the case in which four genes (i.e., the OCT3/4 gene, the SOX2 gene, the c-MYC gene, and the KLF4 gene) were introduced. Thus, the present invention demonstrated for the first time that iPS cells could be established even with the sole use of the OCT3/4 gene in the presence of miRNA.

Fig. 11 shows colony images at the time of colony formation and at the first passage of the iPS cells (i.e. 166G1 and 168G5), which were established via introduction of the OCT3/4 gene and hsa-miR-302-367 cluster miRNA. Also, Fig. 12 shows the results showing that the iPS cells are positive for alkaline phosphatase and thus are in an undifferentiated state.

### INDUSTRIAL APPLICABILITY

The present invention provides an efficient method for preparing induced pluripotent stem cells. The method of the present invention is superior in that it can provide a higher nuclear reprogramming efficiency when compared with conventional methods. Even if the number of reprogramming factors is decreased, induced pluripotent stem cells can be efficiently prepared. The induced pluripotent stem cells prepared by the method of the present invention can be used for inducing differentiated somatic cells. For example, the induced pluripotent stem cells may be converted into therapeutically useful somatic cells, such as myocardial cells, insulin-producing cells, or nerve cells, and such somatic cells can be used for stem cell transplantation therapies for treatment of various diseases, such as heart failure, insulin dependent diabetes, Parkinson's diseases, and spinal cord injury.

## Claims

1. A method for preparing an induced pluripotent stem cell from a somatic cell, comprising a step of nuclear reprogramming of the somatic cell with an exogenous nuclear reprogramming factor(s) in the presence of at least one exogenous miRNA, wherein:
(a) the nuclear reprogramming factor comprises no Sox family member and
(i) Oct3/4;
(ii) Oct3/4 and a Klf family member selected from Klfl, Klf2, Klf4 and Klf5; or
(iii) Oct3/4 and Nanog,
and the miRNA is a miRNA of the hsa-miR-302-367 cluster or a mature miRNA thereof; or
(b) the nuclear reprogramming factor comprises Oct3/4, a Klf family member selected from Klfl, Klf2, Klf4 and Klf5, a Myc family member selected from c-Myc, N-Myc and L-Myc and no Sox family member, and the miRNA is selected from mmu-miR-295/295* , the hsa-miR-302-367 cluster and mature miRNAs thereof; or
(c) the nuclear reprogramming factor comprises Oct3/4, Sox2 and a Klf family member selected from Klfl, Klf2, Klf4 and Klf5, and the miRNA is one or more miRNAs selected from the group consisting of:
- hsa-miR-372;
- hsa-miR-373 or hsa-miR-373/373*;
- hsa-miR-371-373 cluster;
- hsa-miR-302b or hsa-miR-302b/302b*;
- hsa-miR-302-367 cluster;
- hsa-miR-520c or hsa-miR-520c-5p/520c-3p;
- mmu-miR-291a or mmu-miR-291a-5p/291a-3p;
- mmu-miR-294 or mmu-miR-294/294*;
- mmu-miR-295 or mmu-miR-295/295*; and
- mature miRNAs thereof,
where the symbols indicate the miRBase database registration names.

2. A method for increasing the nuclear reprogramming efficiency for inducing an induced pluripotent stem cell from a somatic cell, which method comprises subjecting the somatic cell to nuclear reprogramming with an exogenous nuclear reprogramming factor(s) in the presence of at least one exogenous miRNA, wherein:
(a) the nuclear reprogramming factor comprises no Sox family member and
(i) Oct3/4;
(ii) Oct3/4 and a Klf family member selected from Klfl, Klf2, Klf4 and Klf5; or
(iii) Oct3/4 and Nanog,
and the miRNA is the hsa-miR-302-367 cluster or a mature miRNA thereof; or
(b) the nuclear reprogramming factor comprises Oct3/4, a Klf family member selected from Klf1, Klf2, Klf4 and Klf5, a Myc family member selected from c-Myc, N-Myc and L-Myc and no Sox family member, and the miRNA is selected from mmu-miR-295/295* , the hsa-miR-302-367 cluster and mature miRNAs thereof; or
(c) the nuclear reprogramming factor comprises Oct3/4, Sox2 and a Klf family member selected from Klfl, Klf2, Klf4 and Klf5, and the miRNA is one or more miRNAs selected from the group consisting of:
- hsa-miR-372;
- hsa-miR-373 or hsa-miR-373/373*;
- hsa-miR-371-373 cluster;
- hsa-miR-302b or hsa-miR-302b/302b*;
- hsa-miR-302-367 cluster;
- hsa-miR-520c or hsa-miR-520c-5p/520c-3p;
- mmu-miR-291a or mmu-miR-291a-5p/291a-3p;
- mmu-miR-294 or mmu-miR-294/294*;
- mmu-miR-295 or mmu-miR-295/295*; and
- mature miRNAs thereof,
where the symbols indicate the miRBase database registration names.

3. The method according to claim 1 or 2, wherein the Klf family member is Klf4, and/or the Myc family member is c-Myc.

4. The method according to any one of claims 1 to 3, which comprises introducing a vector comprising a DNA(s) encoding a nuclear reprogramming factor(s) and/or at least one miRNA into the somatic cells.

5. The method according to any one of claims 1 to 3, which comprises introducing a vector comprising a DNA(s) encoding a nuclear reprogramming factor(s) and/or a vector comprising a DNA encoding at least one miRNA or precursor RNA thereof, into the somatic cells.

6. The method according to any one of claims 1 to 5, wherein the miRNA is one or more miRNAs contained in one or more RNA sequences selected from SEQ ID NOS: 1 to 5, 10 to 12, and 14 in the Sequence Listing.

7. The method according to any one of claims 1 to 6, wherein the miRNA comprises 18 to 25 nucleotides.

8. The method according to claim 7, wherein the somatic cell is a human somatic cell.

9. A kit for preparing an induced pluripotent stem cell from a somatic cell, comprising any of the following combinations of components, (a) to (d):
(a) a combination of a miRNA, or a vector encoding the miRNA, with:
(i) Oct3/4, or a vector containing DNA encoding Oct3/4; or
(ii) Oct3/4, or a vector containing DNA encoding Oct3/4, and a Klf family member selected from Klf1, Klf2, Klf4 and Klf5, or a vector containing DNA encoding the Klf family member; or
(iii) Oct3/4, or a vector containing DNA encoding Oct3/4, and Nanog, or a vector containing DNA encoding Nanog;
wherein the miRNA is the hsa-miR-302-367 cluster or mature miRNA(s) thereof comprising 18 to 25 nucleotides; or
(b) a combination of a miRNA, or a vector encoding the miRNA, with Oct3/4, or a vector containing DNA encoding Oct3/4, a Klf family member selected from Klfl, Klf2, Klf4 and Klf5, or a vector containing DNA encoding the Klf family member, and an Myc family member selected from c-Myc, N-Myc and L-Myc, or a vector containing DNA encoding the Myc family member, wherein the miRNA is at least one miRNA selected from the group consisting of: mmu-miR-295/295*, the hsa-miR-302-367 cluster and mature miRNAs thereof comprising 18 to 25 nucleotides; or
(c) a combination of a miRNA, or a vector encoding the miRNA, with Oct3/4, or a vector containing DNA encoding Oct3/4, a Klf family member selected from Klf1, Klf2, Klf4 and Klf5, or a vector containing DNA encoding the Klf family member, and Sox2, or a vector containing DNA encoding Sox2, wherein the miRNA is one or more miRNAs selected from the group consisting of:
- hsa-miR-372;
- hsa-miR-373 or hsa-miR-373/373*;
- hsa-miR-371-373 cluster;
- hsa-miR-302b or hsa-miR-302b/302b*;
- hsa-miR-302-367 cluster;
- hsa-miR-520c or hsa-miR-520c-5p/520c-3p;
- mmu-miR-291a or mmu-miR-291a-5p/291a-3p;
- mmu-miR-294 or mmu-miR-294/294*;
- mmu-miR-295 or mmu-miR-295/295*; and
- mature miRNAs thereof comprising 18 to 25 nucleotides; or
(d) a combination of any of (a)(i) to (iii), (b) and (c) above,
where the symbols indicate the miRBase database registration names.

10. The kit according to claim 9, wherein the Klf family member is Klf4, the Myc family member is c-Myc.

## Patentansprüche

1. Verfahren zur Herstellung einer induzierten pluripotenten Stammzelle aus einer somatischen Zelle, umfassend eine Stufe des nuklearen Reprogrammierens der somatischen Zelle mit einem exogenen nuklearen Reprogrammierungsfaktor(en) in Gegenwart von mindestens einer exogenen miRNA, wobei:
(a) der nukleare Reprogrammierungsfaktor kein Mitglied der Sox-Familie sowie folgendes umfasst
(i) Oct3/4;
(ii) Oct3/4 und ein Mitglied der Klf-Familie, ausgewählt aus Klf1, Klf2, Klf4 und Klf5; oder
(iii) Oct3/4 und Nanog,
und wobei es sich bei der miRNA um eine miRNA des hsa-miR-302-367-Clusters oder um eine reife miRNA davon handelt, oder
(b) der nukleare Reprogrammierungsfaktor folgendes umfasst: Oct3/4, ein Mitglied der Klf-Familie, ausgewählt aus Klf1, Klf2, Klf4 und K1f5, ein Mitglied der Myc-Familie, ausgewählt aus c-Myc, N-Myc und L-Myc, und kein Mitglied der Sox-Familie, und die miRNA ausgewählt ist aus mmu-miR-295/295*, dem hsa-miR-302-367-Cluster und reifen miRNAs davon; oder
(c) der nukleare Reprogrammierungsfaktor Oct3/4, Sox2 und ein Mitglied der Klf-Familie, ausgewählt aus Klf1, Klf2, Klf4 und Klf5, umfasst und es sich bei der miRNa um eine oder mehrere miRNAs handelt, die aus der Gruppe ausgewählt sind die besteht aus:
- hsa-miR-372;
- hsa-miR-373 oder hsa-miR-373/373*;
- hsa-miR-371-373-Cluster;
- hsa-miR-302b oder hsa-miR-302b/302b*;
- hsa-miR-302-367-Cluster;
- hsa-miR-520c oder hsa-miR-520c-5p/520c-3p;
- mmu-miR-291a oder mmu-miR-291a-5p/291a-3p;
- mmu-miR-294 oder mmu-miR-294/294*;
- mmu-miR-295 oder mmu-miR-295/295*; und
- reifen miRNAs davon,
wobei die Symbole die miRBase-Datenbank-Registrierungsnamen angeben.

2. Verfahren zur Erhöhung des nuklearen
Reprogrammierungswirkungsgrads zur Induktion einer induzierten pluripotenten Stammzelle aus einer somatischen Zelle, wobei man bei dem Verfahren die somatische Zelle einer nuklearen Reprogrammierung mit einem exogenen nuklearen Reprogrammierungsfaktor(en) in Gegenwart mindestens einer exogenen miRNA unterwirft, wobei
(a) der nukleare Reprogrammierungsfaktor kein Mitglied der Sox-Familie sowie folgendes umfasst
(i) Oct3/4;
(ii) Oct3/4 und ein Mitglied der Klf-Familie, ausgewählt aus Klf1, Klf2, Klf4 und Klf5; oder
(iii) Oct3/4 und Nanog,
und wobei es sich bei der miRNA um den hsa-miR-302-367-Cluster oder um eine reife miRNA davon handelt, oder
(b) der nukleare Reprogrammierungsfaktor folgendes umfasst: Oct3/4, ein Mitglied der Klf-Familie, ausgewählt aus Klf1, Klf2, Klf4 und Klf5, ein Mitglied der Myc-Familie, ausgewählt aus c-Myc, N-Myc und L-Myc, und kein Mitglied der Sox-Familie, und die miRNA ausgewählt ist aus mmu-miR-295/295*, dem hsa-miR-302-367-Cluster und reifen miRNAs davon; oder
(c) der nukleare Reprogrammierungsfaktor Oct3/4, Sox2 und ein Mitglied der Klf-Familie, ausgewählt aus Klf1, Klf2, Klf4 und Klf5, umfasst und es sich bei der miRNa um eine oder mehrere miRNAs handelt, die aus der Gruppe ausgewählt sind die besteht aus:
- hsa-miR-372;
- hsa-miR-373 oder hsa-miR-373/373*;
- hsa-miR-371-373-Cluster;
- hsa-miR-302b oder hsa-miR-302b/302b*;
- hsa-miR-302-367-Cluster;
- hsa-miR-520c oder hsa-miR-520c-5p/520c-3p;
- mmu-miR-291a oder mmu-miR-291a-5p/291a-3p;
- mmu-miR-294 oder mmu-miR-294/294*;
- mmu-miR-295 oder mmu-miR-295/295*; und
- reifen miRNAs davon,
wobei die Symbole die miRBase-Datenbank-Registrierungsnamen angeben.

3. Verfahren nach Anspruch 1 oder 2, wobei es sich beim Mitglied der Klf-Familie um Klf4 handelt und/oder es sich beim Miglied der Myc-Familie um c-Myc handelt.

4. Verfahren nach einem der Ansprüche 1 bis 3, das die Einführung eines Vektors, der eine oder mehrere DNAs, die für einen oder mehrere nukleare Reprogrammierungsfaktoren kodieren, und/oder mindestens einer miRNA in die somatischen Zellen umfasst.

5. Verfahren nach einem der Ansprüche 1 bis 3, das die Einführung eines Vektors, der eine oder mehrere DNAs, die für einen oder mehrere nukleare Reprogrammierungsfaktoren kodieren, und/oder eines Vektors, der eine DNA umfasst, die für mindestens eine miRNA oder eine Vorläufer-RNA davon kodiert, in die somatischen Zellen umfasst.

6. Verfahren nach einem der Ansprüche 1 bis 5, wobei es sich bei der miRNAs um eine oder mehrere miRNAs handelt, die in einer oder mehreren RNA-Sequenzen enthalten sind, die aus SEQ NO: 1 bis 5, 10 bis 12 und 14 im Sequenzprotokoll ausgewählt sind.

7. Verfahren nach einem der Ansprüche 1 bis 6, wobei die miRNA 18 bis 25 Nucleotide umfasst.

8. Verfahren nach Anspruch 7, wobei es sich bei der somatischen Zelle um eine humane somatische Zelle handelt.

9. Kit zur Herstellung einer induzierten pluripotenten Stammzelle aus einer somatischen Zelle, umfassend eine der folgenden Kombinationen von Komponenten (a) bis (d):
(a) eine Kombination einer miRNA oder eines für die miRNA kodierenden Vektors mit:
(i) Oct3/4 oder einem Vektor, der für Oct3/4 kodierende DNA enthält; oder
(ii) Oct3/4 oder einem Vektor, der für Oct3/4 kodierende DNA enthält, und einem Mitglied der Klf-Familie, ausgewählt aus Klf1, Klf2, Klf4 und Klf5, oder einem Vektor, der für das Mitglied der Klf-Familie kodierende DNA enthält; oder
(iii) Oct3/4 oder einem Vektor, der für Oct3/4 kodierende DNA enthält, und Nanog oder einem Vektor, der für Nanog kodierende DNA enthält;
wobei es sich bei der miRNA um den hsa-miR-302-367-Cluster oder um eine oder mehrere reife miRNAs davon mit 18 bis 25 Nucleotiden handelt; oder
(b) eine Kombination aus einer miRNA oder einem für die miRNA kodierenden Vektor mit Act3/4 oder einem Vektor, der für Act3/4 kodierende DNA enthält, einem Mitglied der Klf-Familie, ausgewählt aus Klf1, Klf2, Klf4 und Klf5, oder einem Vektor, der für das Mitglied der Klf-Familie kodierende DNA enthält, und einem Mitglied der Myc-Familie, ausgewählt aus c-Myc, N-Myc und L-Myc, oder einem Vektor, der für das Mitglied der Myc-Familie kodierende DNA enthält, wobei es sich bei der miRNA um mindestens eine miRNA handelt, die aus der Gruppe ausgewählt ist, die besteht aus: mmu-miR-295/295*, dem hsa-miR-302-367-Cluster und reifen miRNAs davon mit 18 bis 25 Nucleotiden;
(c) einer Kombination aus einer miRNA oder einem für die miRNA kodierenden Vektor mit Oct3/4 oder einem Vektor, der für Oct3/4 kodierende DNA enthält, ein Mitglied der Klf-Familie, ausgewählt aus Klf1, Klf2, Klf4 und Klf5, oder einem Vektor, der für das Mitglied der Klf-Familie kodierende DNA enthält, und Sox2 oder einem Vektor, der für Sox2 kodierende DNA enthält, wobei es sich bei der miRNA um eine oder mehre miRNAs handelt, die aus der Gruppe ausgewählt sind, die besteht aus:
- hsa-miR-372;
- hsa-miR-373 oder hsa-miR-373/373*;
- hsa-miR-371-373-Cluster;
- hsa-miR-302b oder hsa-miR-302b/302b*;
- hsa-miR-302-3&7-Cluster;
- hsa-miR-520c oder hsa-miR-520c-5p/520c-3p;
- mmu-miR-291a oder mmu-miR-291a-5p/291a-3p;
- mmu-miR-294 oder mmu-miR-294/294*;
- mmu-miR-295 oder mmu-miR-295/295*; und
- reifen miRNAs davon mit 18 bis 25 Nucleotiden; oder
(d) eine Kombination aus einem der vorstehenden Abschnitte (a)(i) bis (iii), (b) und (c),
wobei die Symbole die miRBase-Datenbank-Registrierungsnamen angeben.

10. Kit nach Anspruch 9, wobei es sich beim Mitglied der Klf-Familie um Klf4 handelt und es sich beim Mitglied der Myc-Familie um c-Myc handelt.

## Revendications

1. Méthode pour préparer une cellule souche pluripotente induite à partir d'une cellule somatique, comprenant une étape de reprogrammation nucléaire de la cellule somatique avec un ou plusieurs facteurs exogènes de reprogrammation nucléaire en présence d'au moins un miARN, dans laquelle :
(a) le facteur de reprogrammation nucléaire ne comprend aucun membre de la famille Sox et
(i) Oct3/4 ;
(ii) Oct3/4 et un membre de la famille Klf sélectionné parmi Klf1, Klf2, Klf4 et Klf5 ; ou
(iii) Oct3/4 et Nanog,
et le miARN est un miARN du cluster hsa-miR-302-367 ou un miARN mature de celui-ci ; ou
(b) le facteur de reprogrammation nucléaire comprend Oct3/4, un membre de la famille Klf sélectionné parmi Klf1, Klf2, Klf4 et Klf5, un membre de la famille Myc sélectionné parmi c-Myc, N-Myc et L-Myc et aucun membre de la famille Sox, et le miARN est sélectionné parmi mmu-miR-295/295*, le cluster hsa-miR-302-367 et les miARN matures de ceux-ci ; ou
(c) le facteur de reprogrammation nucléaire comprend Oct3/4, Sox2 et un membre de la famille Klf sélectionné parmi Klf1, Klf2, Klf4 et Klf5, et le miARN est un ou plusieurs miARN sélectionnés dans le groupe constitué de :
- hsa-miR-372 ;
- hsa-miR-373 ou hsa-miR-373/373* ;
- le cluster hsa-miR-371-373 ;
- hsa-miR-302b ou hsa-miR-302b/302b* ;
- le cluster hsa-miR-302-367 ;
- hsa-miR-520c ou hsa-miR-520c-5p/520c-3p ;
- mmu-miR-291a ou mmu-miR-291a-5p/291a-3p ;
- mmu-miR-294 ou mmu-miR-294/294* ;
- mmu-miR-295 ou mmu-miR-295/295* ; et
- des miARN matures de ceux-ci,
où les symboles indiquent les noms d'enregistrement de la base de données miRBase.

2. Méthode pour augmenter l'efficacité de la reprogrammation nucléaire pour induire une cellule souche pluripotente induite à partir d'une cellule somatique, laquelle méthode comprend la soumission de la cellule somatique à une reprogrammation nucléaire avec un ou plusieurs facteurs exogènes de reprogrammation nucléaire en présence d'au moins un miARN exogène, dans laquelle :
(a) le facteur de reprogrammation nucléaire ne comprend aucun membre de la famille Sox et
(i) Oct3/4 ;
(ii) Oct3/4 et un membre de la famille Klf sélectionné parmi Klf1, Klf2, Klf4 et Klf5 ; ou
(iii) Oct3/4 et Nanog,
et le miARN est le cluster hsa-miR-302-367 ou un miARN mature de celui-ci ; ou
(b) le facteur de reprogrammation nucléaire comprend Oct3/4, un membre de la famille Klf sélectionné parmi Klfl, Klf2, Klf4 et Klf5, un membre de la famille Myc sélectionné parmi c-Myc, N-Myc et L-Myc et aucun membre de la famille Sox, et le miARN est sélectionné parmi mmu-miR-295/295*, le cluster hsa-miR-302-367 et les miARN matures de ceux-ci ; ou
(c) le facteur de reprogrammation nucléaire comprend Oct3/4, Sox2 et un membre de la famille Klf sélectionné parmi Klfl, Klf2, Klf4 et Klf5, et le miARN est un ou plusieurs miARN sélectionnés dans le groupe constitué de :
- hsa-miR-372 ;
- hsa-miR-373 ou hsa-miR-373/373* ;
- le cluster hsa-miR-371-373 ;
- hsa-miR-302b ou hsa-miR-302b/302b* ;
- le cluster hsa-miR-302-367 ;
- hsa-miR-520c ou hsa-miR-520c-5p/520c-3p ;
- mmu-miR-291a ou mmu-miR-291a-5p/291a-3p ;
- mmu-miR-294 ou mmu-miR-294/294* ;
- mmu-miR-295 ou mmu-miR-295/295* ; et
- des miARN matures de ceux-ci,
où les symboles indiquent les noms d'enregistrement de la base de données miRBase.

3. Méthode selon les revendications 1 ou 2, dans laquelle le membre de la famille Klf est Klf4, et/ou le membre de la famille Myc est c-Myc.

4. Méthode selon l'une quelconque des revendications 1 à 3, qui comprend l'introduction d'un vecteur comprenant un ou plusieurs ADN codant pour un ou plusieurs facteurs de reprogrammation nucléaire et/ou au moins un miARN dans les cellules somatiques.

5. Méthode selon l'une quelconque des revendications 1 à 3, qui comprend l'introduction d'un vecteur comprenant un ou plusieurs ADN codant pour un ou plusieurs facteurs de reprogrammation nucléaire et/ou un vecteur comprenant un ADN codant pour au moins un miARN ou un ARN précurseur de celui-ci, dans les cellules somatiques.

6. Méthode selon l'une quelconque des revendications 1 à 5, dans laquelle le miARN est un ou plusieurs miARN contenus dans une ou plusieurs séquences d'ARN sélectionnées parmi SÉQ ID NO : 1 à 5, 10 à 12, et 14 dans la liste des séquences.

7. Méthode selon l'une quelconque des revendications 1 à 6, dans laquelle le miARN comprend 18 à 25 nucléotides.

8. Méthode selon la revendication 7, dans laquelle la cellule somatique est une cellule somatique humaine.

9. Kit pour préparer une cellule souche pluripotente induite à partir d'une cellule somatique, comprenant l'une quelconque des combinaisons suivantes de composants, (a) à (d) :
(a) une combinaison d'un miARN, ou d'un vecteur codant pour le miARN, avec :
(i) Oct3/4, ou un vecteur contenant l'ADN codant pour Oct3/4 ; ou
(ii) Oct3/4, ou un vecteur contenant l'ADN codant pour Oct3/4, et un membre de la famille Klf sélectionné parmi Klf4, Klf2, Klf4 et Klf5, ou un vecteur contenant l'ADN codant pour le membre de la famille Klf ; ou
(iii) Oct3/4, ou un vecteur contenant l'ADN codant pour Oct3/4, et Nanog, ou un vecteur contenant l'ADN codant pour Nanog ;
dans lequel le miARN est le cluster hsa-miR-302367 ou des miARN matures de celui-ci comprenant 18 à 25 nucléotides ; ou
(b) une combinaison d'un miARN, ou d'un vecteur codant pour le miARN, avec Oct3/4, ou un vecteur contenant l'ADN codant pour Oct3/4, un membre de la famille Klf sélectionné parmi Klf1, Klf2, Klf4 et Klf5, ou un vecteur contenant l'ADN codant pour le membre de la famille Klf, et un membre de la famille Myc sélectionné parmi c-Myc, N-Myc et L-Myc, ou un vecteur contenant l'ADN codant pour le membre de la famille Myc, dans lequel le miARN est au moins un miARN sélectionné dans le groupe constitué de : mmu-miR-295/295*, le cluster hsamiR-302-367 et les miARN matures de ceux-ci comprenant 18 à 25 nucléotides ; ou
(c) une combinaison d'un miARN, ou d'un vecteur codant pour le miARN, avec Oct3/4, ou un vecteur contenant l'ADN codant pour Oct3/4, un membre de la famille Klf1, Klf2, Klf4 et Klf5, ou un vecteur contenant l'ADN codant pour le membre de la famille Klf, et Sox2, ou un vecteur contenant l'ADN codant pour Sox2, dans lequel le miARN est un ou plusieurs miARN sélectionnés dans le groupe constitué de :
- hsa-miR-372 ;
- hsa-miR-373 ou hsa-miR-373/373* ;
- le cluster hsa-miR-371-373 ;
- hsa-miR-302b ou hsa-miR-302b/302b* ;
- le cluster hsa-miR-302-367 ;
- hsa-miR-520c ou hsa-miR-520c-5p/520c-3p ;
- mmu-miR-291a ou mmu-miR-291a-5p/291a-3p ;
- mmu-miR-294 ou mmu-miR-294/294* ;
- mmu-miR-295 ou mmu-miR-295/295* ; et
- des miARN matures de ceux-ci comprenant 18 à 25 nucléotides ; ou
(d) une combinaison de l'un quelconque de (a)(i) à (iii), (b) et (c) ci-dessus,
où les symboles indiquent les noms d'enregistrement de la base de données miRBase.

10. Kit selon la revendication 9, dans lequel le membre de la famille Klf est Klf4, le membre de la famille Myc est c-Myc.
